# EUROPEAN PATENT SPECIFICATION

(11) **EP 0 649 843 B1**
(45) Date of publication and mention of the grant of the patent: **06.09.2000**
(21) Application number: 93914969.6
(22) Date of filing: 08.07.1993
(51) Int. Cl.: C07D 277/56, C07D 417/12, A61K 31/425, A61K 31/44

(54) **THIAZOLINE DERIVATIVE**
THIAZOLIN-DERIVATE
DERIVE DE THIAZOLINE

(30) Priority: 15.07.1992 JP 18833592; 27.11.1992 JP 31840292
(43) Date of publication of application: 26.04.1995
(73) Proprietor: TAISHO PHARMACEUTICAL CO. LTD, Tokyo 171 (JP)
(72) Inventor: SATO, Masakazu, Taisho Pharmaceutical Co. Ltd., Toshima-ku, Tokyo 171 (JP); MANAKA, Akira, Taisho Pharmaceutical Co. Ltd., Toshima-ku, Tokyo 171 (JP); TAKAHASHI, Keiko, Taisho Pharmaceutical Co. Ltd., Toshima-ku, Tokyo 171 (JP); KAWASHIMA, Yutaka, Taisho Pharmaceutical Co. Ltd., Toshima-ku, Tokyo 171 (JP); HATAYAMA, Katsuo, Taisho Pharmaceutical Co. Ltd., Toshima-ku, Tokyo 171 (JP)
(74) Representative: Kraus, Walter, Dr.
(86) International application number: JP9300937
(87) International publication number: WO9402472

(56) References cited:
- EP-A- 0 261 503
- JP-A- 2 050 148

## Description

### TECHNICAL FIELD

The present invention relates to thiazoline derivatives, and more particularly to thiazoline derivatives having fibrinogen receptor antagonism and cell adhesion factor antagonism.

### BACKGROUND ART

Blood platelets are considered to bind each other via fibrinogen and aggregate as a result of the appearance of the binding site of fibrinogen on the blood platelet membrane glycoprotein GPIIb/IIIa complex caused by stimulation of various blood platelet aggregation-inducers. Therefore, the compounds having the antagonism against fibrinogen receptor are expected to have the inhibitory action of blood platelet aggregation. Among the known compounds having the inhibitory action of blood platelet aggregation are peptide derivatives such as Arg-Gly-Asp-Ser containing Arg-Gly-Asp (hereinafter referred to as RGDS) which is considered to be a binding site of fibrinogen receptor [Thrombosis Res., vol. 56, No. 6, page 687 (1989)] and compounds having an amidino group in the molecule (described in Japanese Patent Kokai 2-223543).

However, the above compounds are insufficiently effective.

An object of the present invention is to provide compounds having excellent fibrinogen receptor antagonism and cell adhesion factor antagonism, i.e., excellent inhibitory agents for blood platelet aggregation.

### DISCLOSURE OF THE INVENTION

As a result of various researches, the present inventors have found thiazoline derivatives having fibrinogen receptor antagonism and cell adhesion factor antagonism, and have accomplished the present invention.

The present invention is a thiazoline derivative represented by the formula: 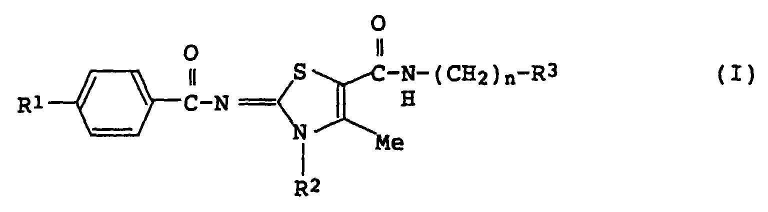 [wherein R¹ is a cyano group, a carbamoyl group, a thiocarbamoyl group, a morpholinothiocarbonyl group, an alkylthioimidoyl group having 2 to 7 carbon atoms, a group represented by the formula: 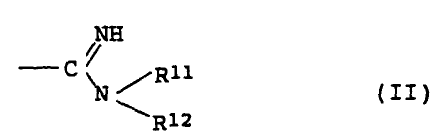 (wherein R¹¹ and R¹² are the same or different and are each a hydrogen atom, an alkyl group having 1 to 6 carbon atoms, an alkoxycarbonyl group having 2 to 7 carbon atoms, a cycloalkyl group having 4 to 8 carbon atoms, a phenyl group, a phenyl group substituted by an alkyl group having 1 to 4 carbon atoms, an alkoxy group having 1 to 4 carbon atoms or a halogen atom, an aralkyl group being an alkyl group having 1 to 3 carbon atoms substituted by a phenyl or a naphthyl group at the end thereof, or the aralkyl group substituted by an alkyl group having 1 to 4 carbon atoms, an alkoxy group having 1 to 4 carbon atoms, a trifluoromethyl group or a halogen atom or R¹¹ and R¹² together with the adjacent nitrogen atom form a univalent group of a 4- to 7-membered heterocyclic ring having at least one nitrogen atom on the ring which may have a substituent selected from alkyl, phenyl or benzyl at the 4-position) or an imidazolin-2-yl group; n is an integer of 1 to 3, R² is an alkyl group having 1 to 10 carbon atoms or an aralkyl group being an alkyl group having 1 to 3 carbon atoms substituted by a phenyl or a naphthyl group at the end thereof, and R³ is a carboxyl group, a hydroxyl group, an alkoxycarbonyl group having 2 to 7 carbon atoms, a benzyloxycarbonyl group or a pyridylmethyloxycarbonyl group] or a salt thereof.

In the present invention, the alkyl group, the alkoxy group and the alkoxycarbonyl group are those which are straight or branched chain. The aralkyl group refers to an alkyl group having 1 to 3 carbon atoms substituted by an aryl group (e.g. a phenyl group or a naphthyl group) at the end thereof such as, for example, benzyl group, a phenethyl group or a naphthylmethyl group. The heterocyclic compound refers to a 4- to 7-membered heterocyclic ring having at least one nitrogen atom on the ring, and examples of a univalent group thereof are a piperidino group, a 1-pyrrolidinyl group, a morpholino group and a 1-piperazinyl group which may have a substituent (e.g. an alkyl group, a phenyl group or a benzyl group) at the 4-position.

The salt of the compounds of Formula (I) refers to salts with alkali metals, alkaline earth metals, ammonia, alkylamines, mineral acids, carboxylic acids or sulfonic acids such as, for example, sodium salt, potassium salt, calcium salt, ammonium salt, aluminium salt, triethylammonium salt, hydrochloride, hydrobromide, hydroiodide, sulfate, nitrate, phosphate, acetate, fumarate, maleate or methanesulfonate.

Among the preferred compounds of the present invention are the compounds wherein R¹ is a group of Formula (II), n is 2, and R³ is other than a hydroxyl group.

The compounds of the present invention can be prepared, for example, by the following methods.

First, a compound represented by the formula; 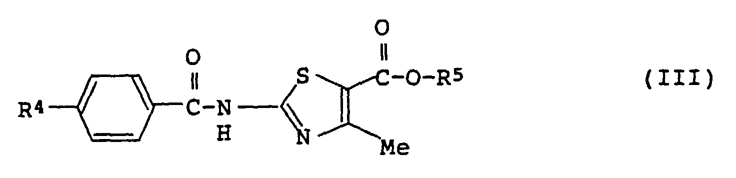 (wherein R⁴ is a cyano group or a morpholinothiocarbonyl group, as defined for R¹, and R⁵ is a lower alkyl group, a benzyl group or a 2-cyanoethyl group) is reacted with a halide (e.g. methyl iodide or benzyl chloride) represented by the formula:

R²-X

(wherein R² is as defined above, and X is a halogen atom) in the presence of a base to lead to a compound represented by the formula: 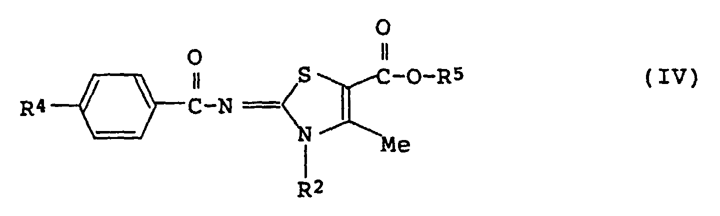 (wherein R², R⁴ and R⁵ are as defined above).

The compound of Formula (IV) wherein R² is other than an aralkyl group can also be obtained by a reaction of an alkylating agent such as a dialkyl sulfate (e.g. dimethyl sulfate) represented by the formula:

R⁶₂-SO₄

(wherein R⁶ is R² other than an aralkyl group), or a sulfonate (e.g. methyl methanesulfonate) represented by the formula:

R⁷SO₃R⁶

(wherein R⁷ is an alkyl group or an aryl group, and R⁶ is as defined above) with a compound of Formula (III).

Next, the ester moiety of the compound of Formula (IV) is hydrolyzed by using a conventional method to lead to a compound represented by the formula: 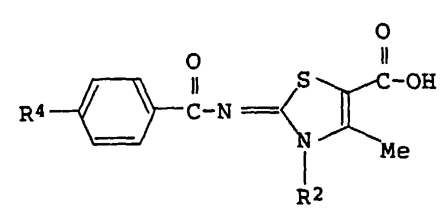 (wherein R² and R⁴ are as defined above) or a salt thereof, and reacted using an amine represented by the formula:

H₂N-(CH₂)ₙ-R³

(wherein n and R³ are as defined above) according to a conventional amide linkage formulation method to give a compound of the present invention of Formula (I) wherein R¹ is a cyano group or a morpholinothiocarbonyl group.

The compound of the present invention wherein R¹ is a cyano group is subjected, for example, to a reaction with hydrogen sulfide by using a base as a catalyst, or a reaction with NaBH₂S₃ to give a compound of the present invention wherein R¹ is a thiocarbamoyl group. The compound of the present invention wherein R¹ is a cyano group can be reacted with hydrogen peroxide in the presence of a base according to an ordinary method to lead to a compound of the present invention wherein R¹ is a carbamoyl group.

The resulting compound of the present invention wherein R¹ is a thiocarbamoyl group is treated with a lower alkyl halide represented by the formula:

R⁸-X

(wherein R⁸ is a lower alkyl group, and X is a halogen atom) to give a compound of the present invention wherein R¹ is an alkylthioimidoyl group having 2 to 7 carbon atoms, and reacted with ammonia, a monoalkylamine having 1 to 6 carbon atoms, a dialkylamine having 1 to 6 carbon atoms, a monocycloalkylamine having 4 to 8 carbon atoms, aniline, a substituted aniline, an aralkylamine, a substituted aralkylamine, a heterocyclic compound having one or more secondary nitrogen atoms on the ring, 1,2-diaminoethane or a salt thereof to lead to a compound of the present invention wherein R¹ is a group of Formula (II) (wherein R¹¹ or R¹² is other than an alkoxycarbonyl group having 2 to 7 carbon atoms) or a imidazolin-2-yl group.

The compound of the present invention wherein R¹ is a group of Formula (II), R¹¹ and/or R¹² is an alkoxycarbonyl group having 2 to 7 carbon atoms can be obtained by a reaction of the above-resulting compound of the present invention wherein R¹ is a group of Formula (II), and R¹¹ and R¹² are other than an alkoxycarbonyl group having 2 to 7 carbon atoms [hereinafter referred to as a compound of Formula (Ia)] with a compound represented by the formula:

R⁹-O(C=O)-X or

R⁹-O(C=O)-O-(C=O)O-R⁹

(wherein R⁹ is a lower alkyl group, and X is a halogen atom) in the presence of a base.

Alternatively, the compound of the present invention wherein R³ is a carboxyl group or a salt thereof can be also prepared by an ester hydrolysis of the compound of Formula (I) wherein R³ is other than a carboxyl group or a hydroxyl group. The ester hydrolysis to be used is an ordinary method such as an alkali treatment or a mineral acid treatment.

The compound of the present invention wherein R³ is an alkoxycarbonyl group having 2 to 7 carbon atoms, a benzyloxycarbonyl group or a pyridylmethyloxycarbonyl group can be prepared by reacting a compound of the present invention wherein R³ is a carboxyl group or a salt thereof with a compound represented by the formula:

R¹⁰-OH or

R¹⁰-X

(wherein R¹⁰ is a lower alkyl group, a benzyl group or a pyridylmethyl group, and X is a halogen atom) under ordinary conditions of esterification of a carboxylic acid, for example, by treating with dicyclohexyl-carbodiimide in the presence of 4-dimethylaminopyridine. The compound of the present invention wherein R³ is an alkoxycarbonyl group having 2 to 7 carbon atoms, a benzyloxycarbonyl group or a pyridylmethyloxycarbonyl group can be also prepared by interchanging under ordinary ester interchange conditions, for example, by treating with an acid.

The compound of Formula (Ia) can be also synthesized from the compound of the present invention wherein R¹ is a cyano group by using an ordinary method for conversion of a cyano group to an amidino group via iminochloride or imino ether.

The compound of the present invention wherein R¹ is an imidazolin-2-yl group can be also prepared by reacting a compound of the present invention wherein R¹ is a morpholinothiocarbonyl group with a lower alkyl halide represented by the formula;

R⁸-X

(wherein R⁸ and X are as defined above), and reacting the resulting compound with 1,2-diaminoethane or a salt thereof.

Examples of the base to be used in the above reactions are alkaline metal salts (e.g. sodium hydroxide, potassium hydroxide, dimsylsodium, sodium hydride, sodium amide or potassium tert-butoxide), amines (e.g. triethylamine, diisopropylethylamine or pyridine) and a salt thereof (e.g. organic acid salts such as ammonium acetate).

Examples of the reaction solvent to be used are reaction-inert solvents such as water, alcohols (e.g. methanol, ethanol, isopropyl alcohol or tert-butyl alcohol), ethers (e.g. dioxane or tetrahydrofuran), dimethylformamide, dimethyl sulfoxide, pyridine, methylene chloride, chloroform and acetone.

### INDUSTRIAL UTILIZATION

The thus-obtained compounds of Formula (I) inhibit the binding of various adhesive proteins such as fibrinogen, fibronectin or von Willebrand factor against fibrinogen receptor (GpIIb/IIIa) on blood platelet, and have the inhibitory action of the aggregation and adhesion of blood platelet. Additionally, the compounds of Formula (I) inhibit the binding of the above-mentioned adhesive proteins and other adhesive proteins such as vitronectin and collagen which form intercellular matrix on various cell surfaces, and act on biotaxis and cell - intercellular interaction.

Accordingly, the compounds of the present invention can be used as preventive or therapeutic agents for ischemic diseases (e.g. thrombosis, cerebral infarction or myocardial infarction) and atherosclerosis diseases, or metastasis inhibitory agents for malignant tumors.

For the purposes, the compounds of Formula (I) are mixed with, for example, conventional fillers, binders, disintegrators, lubricants, pH moderators and solubilizers, and prepared in forms such as tablets, pills, capsules, granules, powders, solutions, emulsions, suspensions or injectional solutions by conventional pharmaceutical techniques, which can be administered orally or parenterally.

The dose for adult patients is 1 to 1000 mg in the case of oral administration, and 0.01 to 100 mg in the case of parenteral administration, which can be administered in a single dose or in several divided doses per day. This dose can be increased or reduced depending on the kind of the diseases and the age, body weight and condition of the patient.

The effect of the compounds of Formula (I) is illustrated by the following experiments.

### Experiment 1 [Human blood platelet fibrinogen binding test]

Citrated blood (the volume ratio of 3.13% sodium citrate solution and blood is 1:9) was collected from the cubital vein of a healthy human who had not received any drugs known to affect the function of blood platelet within 2 weeks prior to starting the test, and was centrifuged at 120 x g at room temperature for 15 minutes to give platelet rich plasma (PRP) as a supernatant.

To the above PRP was added one fifth volume of an ACD solution (citric acid : sodium citrate : dextrose = 68.7 mM : 85 mM : 11.1 mM), followed by centrifugation at 1200 x g for 15 minutes. The precipitate was suspended in a Tyrode's solution (20% fetal bovine serum, 2 mM Mg²⁺), followed by gel filtration using Sepharose 2B column to give a blood platelet suspension (1 x 10⁹/ml) apart from fibrinogen. The binding test was carried out by using the blood platelet suspension apart from fibrinogen, a solution of the compound of Formula I as a test drug in dimethyl sulfoxide which was adjusted to the desired concentration by diluting with a physiological saline solution, ADP (final concentration: 10 µM), and ¹²⁵I labelled human fibrinogen. The binding inhibition rate of the test drug was then calculated.

RGDS (produced by Sigma Co.) and 3-[3-(4-amidinobenzoyl)benzamide]propionic acid (described in Japanese Patent Kokai 2-223543; hereinafter referred to as "control") were used as comparative drugs, and the test solutions of these drugs were prepared in the same manner as described above, and tested as described above.

The results are shown in Table 1 in which the compound numbers are as defined in the following examples.

**Table 1**

| Compound No. | IC₅₀ (nM) |
|---|---|
| Compound 5 | 5.1 |
| Compound 7 | 2.7 |
| Compound 23 | 6.3 |
| Compound 29 | 7.9 |
| Compound 35 | 1.0 |
| Compound 37 | 1.3 |
| Control | 84.4 |
| RGDS | 180000 |

### Experiment 2[Inhibition Test of Human In Vitro Platelet Aggregation]

Citrated blood (the volume ratio of 3.13% sodium citrate solution and blood is 1:9) was collected from the cubital vein of a healthy human who had not received any drugs known to affect the function of blood platelet within 2 weeks prior to starting the test, and was centrifuged at 120 x g at room temperature for 15 minutes to give platelet rich plasma (PRP) as a supernatant, and was centrifuged at 1500 x g for 10 minutes to give platelet poor plasma (ppp). The blood platelet counts of PRP were adjusted to (50 to 60) x 10⁴/µl by diluting with PPP.

Blood platelet aggregation was determined according to the method of Born G.V.R., [Nature, vol. 194, page 927 (1962)] using adenosine diphosphate (produced by Sigma Co.; hereinafter referred to as "ADP") as an aggregation-inducer. That is, a solution of the compound of Formula (I) as a test drug in dimethyl sulfoxide was adjust to the desired concentration with a physiological saline solution. 25 µl of the solution was added to 250 µl of PRP and incubated at 37°C for 3 minutes, and 25 µl of ADP (final concentration : 7 µM) was added thereto. The mixture was measured for 5 minutes by using a blood platelet aggregation ability measurement apparatus (Aggricoda TM·PA-3210; made by Kyoto Daiichi Kagaku Co.) to give the maximum aggregation, and the concentration of the test drug to cause 50% inhibition of the maximum aggregation (IC₅₀) was calculated.

Results are shown in Table 2 wherein the compound numbers are as defined in the following examples.

**Table 2**

| Compound No. | IC₅₀ (nM) |
|---|---|
| Compound 5 | 17 |
| Compound 17 | 19 |
| Compound 22 | 18 |
| Compound 27 | 15 |
| Compound 29 | 14 |
| Compound 31 | 20 |
| Control | 105 |

### BEST MODE FOR CARRYING OUT THE INVENTION

### Example 1

(1) A mixture of 4-cyanobenzoyl chloride (166.5 g), ethyl 2-amino-4-methylthiazole-5-carboxylate hydrochloride (224.0 g) and pyridine (2000 ml) was stirred at room temperature for 70 minutes. The precipitated crystals were collected by filtration, and washed with 30% hydrochloric acid and water to give ethyl 2-(4-cyanobenzoylamino)-4-methylthiazole-5-carboxylate (262.0 g).
   m.p. 293 - 295°c.
(2) The compound obtained in (1) (22.07 g) was added to a suspension of 60% sodium hydride in oil (3.08 g) in N,N-dimethylformamide (hereinafter referred to as "DMF") (300 ml) under ice-cooling, followed by stirring at room temperature for an hour. A solution of methyl iodide (4.8 ml) in DMF (50 ml) was added dropwise to the reaction mixture, followed by further stirring at room temperature for an hour. The reaction mixture was taken up in 3% hydrochloric acid, the precipitated crystals were collected by filtration, and the resulting crude crystals were recrystallized from a mixture of methylene chloride and methanol to give ethyl 2-(4-cyanobenzoylimino)-3,4-dimethyl-3H-thiazoline-5-carboxylate (15.97 g).
   m.p. 244 - 245°C
(3) 10% Aqueous sodium hydroxide solution (48 ml) was added to a mixture of the compound obtained in (2) (9.88 g), methylene chloride (250 ml) and methanol (250 ml), followed by stirring at room temperature for 17 hours. The reaction mixture was concentrated under reduced pressure, and the precipitated crystals were collected by filtration to give sodium 2-(4-cyanobenzoylimino)-3,4-dimethyl-3H-thiazoline-5-carboxylate (10.0 g).
   1H-NMR(DMSO-d₆) δ (ppm):
      2.66 (3H, s), 3.75 (3H, s), 7.91 (2H, d, J=8Hz), 8.33 (2H, d, J=8Hz)
(4) β-Alanine methyl ester hydrochloride (4.68 g), 1-hydroxybenzotriazole monohydrate (9.34 g) and 1-ethyl-3-[3-(dimethylamino)propyl]-carbodiimide hydrochloride (6.43 g) were successively added with stirring to a suspension of the compound obtained in (3) (9.85 g) in DMF, followed by stirring at room temperature for 14 hours. The reaction mixture was taken up in water, and the precipitated crystals were collected by filtration, and recrystallized from a mixture of methylene chloride and hexane to give N-(2-methoxycarbonylethyl)-2-(4-cyanobenzoylimino)-3,4-dimethyl-3H-thiazoline-5-carboxamide (Compound 1) (9.9 g).
   m.p. 187.5 - 189.5°C

### Example 2

Hydrogen sulfide was passed through a mixture of Compound 1 (9.66 g), pyridine (500 ml) and triethylamine (8.7 ml) at room temperature with stirring for 3 hours, followed by standing for 16 hours. The reaction mixture was evaporated under reduced pressure, and the resulting crude crystals were washed with ethyl acetate to give N-(2-methoxycarbonylethyl)-2-(4-thiocarbamoylbenzoylimino)-3,4-dimethyl-3H-thiazoline-5-carboxamine (Compound 2) (10.56 g).
m.p. 215.5 -216.5°C

### Example 3

Methyl iodide (28 ml) was divided into 4 portions, which were added in turn to a suspension of Compound 2 (6.31 g) in acetone (1600 ml) under heating reflux at intervals of 30 minutes, followed by stirring for 4 hours. The reaction mixture was concentrated under reduced pressure, and the precipitated crystals were collected by filtration to give N-(2-methoxycarbonylethyl)-2-[4-(methylthioimidoyl)-benzoylimino]-3,4-dimethyl-3H-thiazoline-5-carboxamide hydroiodide (Compound 3) (7.69 g).
m.p. 203.5 - 204°C

### Example 4

A mixture of Compound 3 (7.31 g), ammonium acetate (4.0 g) and methanol (150 ml) was heated under reflux with stirring for 70 minutes. The reaction mixture was concentrated under reduced pressure, and the precipitated crystals were collected by filtration to give N-(2-methoxycarbonylethyl)-2-(4-amidinobenzoylimino)-3,4-dimethyl-3H-thiazoline-5-carboxamide acetate (Compound 4) (4.96 g).
m.p. 223 - 224.5°C

### Example 5

A mixture of Compound 4 (100 mg), water (0.5 ml) and 47% aqueous HBr solution (0.5 ml) was stirred at 80°C for 1.5 hours. The reaction mixture was ice-cooled, and the precipitated crystals were collected by filtration to give N-(2-carboxyethyl)-2-(4-amidinobenzoylimino)-3,4-dimetylthiazoline-5-carboxamide hydrobromide (Compound 5) (63 mg).
m.p. 272 - 273.5°C

### Example 6

Compound 3 (1.12 g) was added to a mixture of 40% methylamine solution in methanol (2.0 ml), methanol (15 ml) and acetic acid (1.2 ml), followed by heating under reflux with stirring for 1.5 hours. The reaction mixture was evaporated under reduced pressure, and the resulting residue was washed with methanol to give N-(2-methoxycarbonylethyl)-2-[4-(N-methylamidino)benzoylimino]-3,4-dimethyl-3H-thiazoline-5-carboxamide hydroiodide (Compound 6) (0.84 g).
m.p. 225 - 227°C

### Example 7

Following the procedure similar to that of Example 5 using Compound 6 (417 mg), there was obtained N-(2-carboxyethyl)-2-[4-(N-methylamidino)benzoylimino]-3,4-dimethyl-3H-thiazoline-5-carboxamide hydrobromide (Compound 7) (289 mg).
¹H-NMR (DMSO-d₆) δ (ppm):
   2.50 (2H, t, J=6Hz), 2.61 (3H, s), 3.03 (3H, d, J=5Hz), 3.43 (2H, q, J=6Hz), 3.85 (3H, s), 7.85 (2H, d, J=8Hz), 8.30 (1H, t, J=6Hz), 8.38 (2H, d, J=8Hz), 9.02 (1H, brs), 9.55 (1H, brs), 9.88 (1H, d, J=5Hz), 12.3 (1H, brs)

### Example 8

Following the procedure similar to that of each of Examples 1 to 5, there were obtained the compounds shown in Table 3 from the corresponding starting materials.

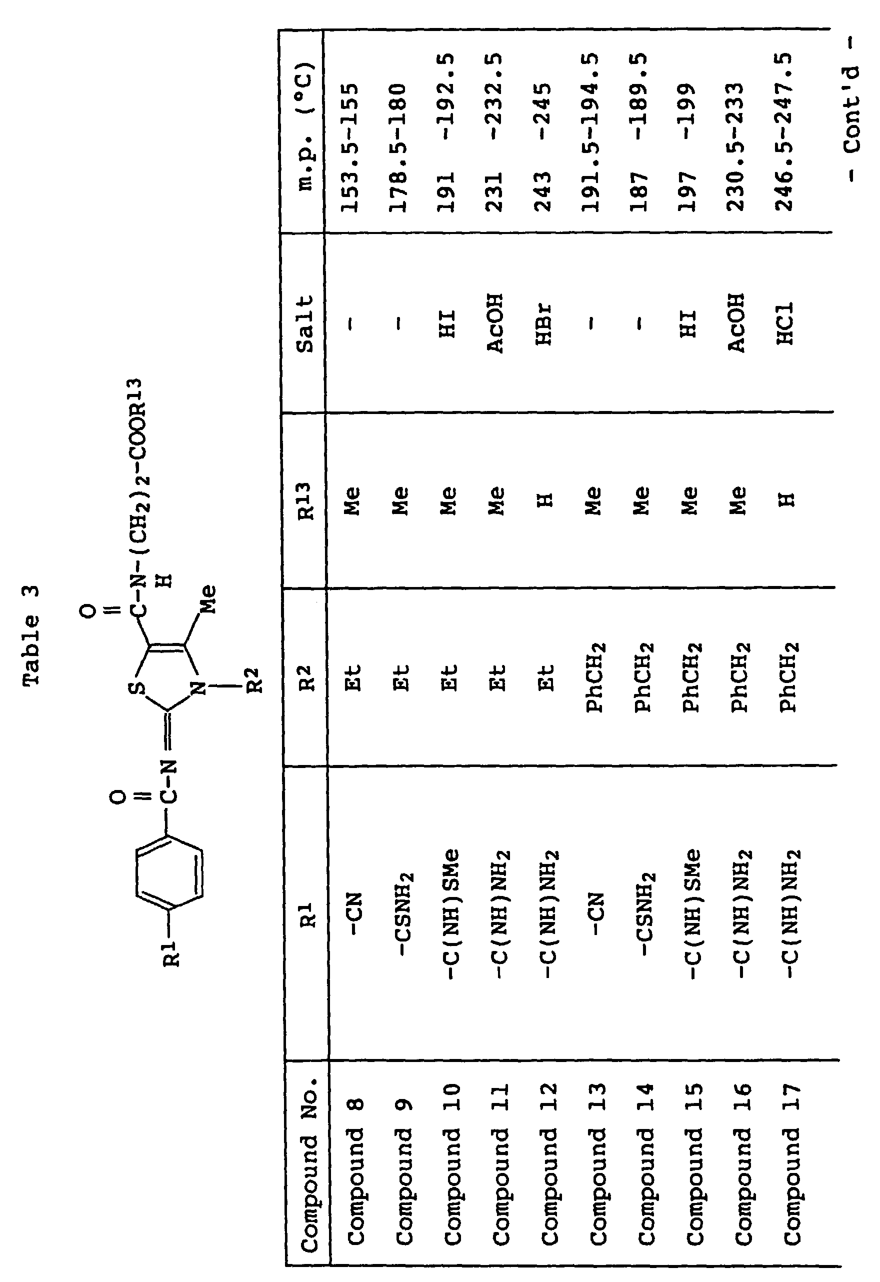

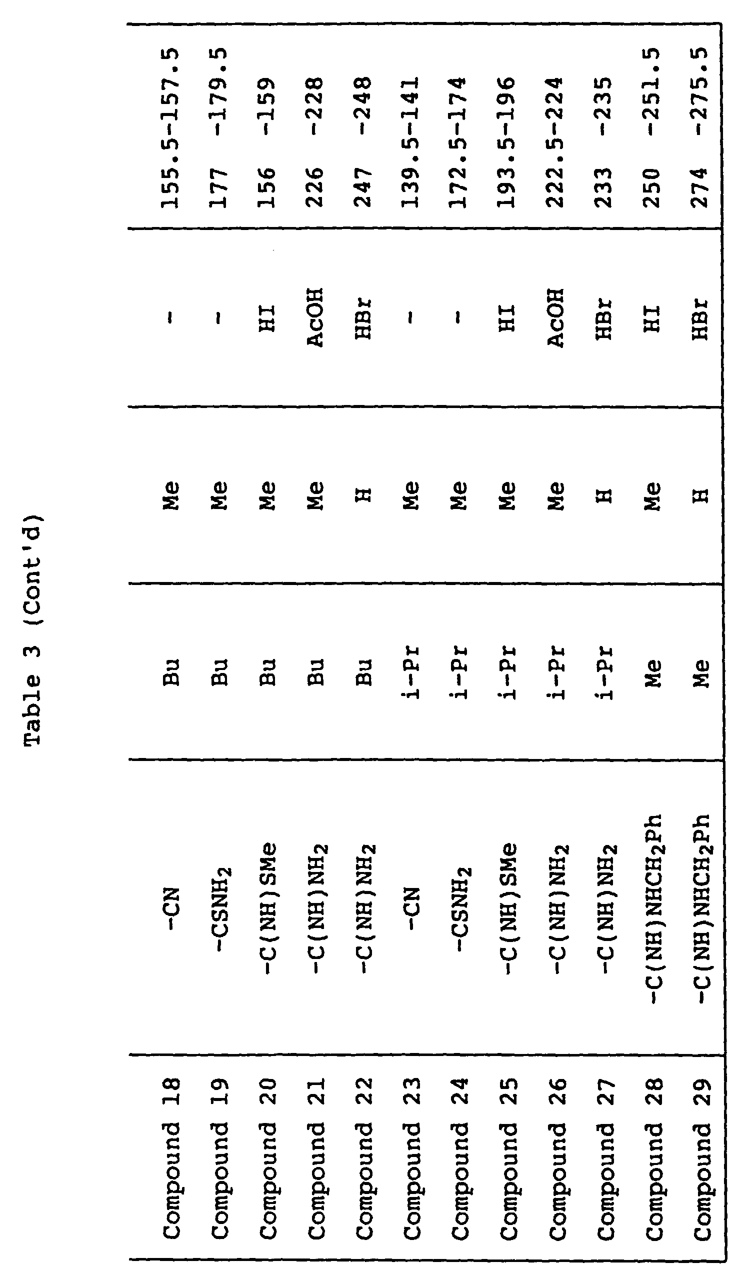

### Example 9

Compound 3 (1.0 g) was added to a mixture of aniline (0.18 ml) and methanol (20 ml), followed by heating under reflux with stirring for 1.5 hours. The reaction mixture was evaporated under reduced pressure, and the resulting residue was washed with methanol to give N-(2-methoxycarbonylethyl)-2-[4-(N-phenylamidino)-benzoylimino]-3,4-dimethyl-3H-thiazoline-5-carboxamide hydroiodide (Compound 30) (0.68 g).
m.p. 211 - 212°C (decomposition)

### Example 10

Following the procedure similar to that of Example 5 using Compound 30, there was obtained N-(2-carboxyethyl)-2-[4-(N-phenylamidino)benzoylimino]-3,4-dimethyl-3H-thiazoline-5-carboxamide hydrobromide (Compound 31).
m.p. 202 - 204°C

### Example 11

A mixture of Compound 4 (0.463 g), methanesulfonic acid (0.1 ml) and ethanol (20 ml) was heated under reflux for 6 hours. The reaction mixture was allowed to stand for cooling, and the precipitated crystals were collected by filtration to give N-(2-ethoxycarbonylethyl)-2-(4-amidinobenzoylimino)-3,4-dimethyl-3H-thiazoline-5-carboxamide methanesulfonate (Compound 32) (0.408 g).
m.p. 252 - 253°C

### Example 12

A mixture of Compound 4 (0.463 g), methanesulfonic acid (0.1 ml) and 2-propanol (20 ml) was heated under reflux for 50 hours. The reaction mixture was allowed to stand for cooling, and the precipitated crystals were collected by filtration to give N-(2-isopropoxycarbonylethyl)-2-(4-amidinobenzoylimino)-3,4-dimethyl-3H-thiazoline-5-carboxamide methanesulfonate (Compound 33) (0.42 g).
m.p. 253 - 253.5°C

### Example 13

A mixture of Compound 3 (1.0 g), N-methylbenzylamine (1.15 ml), acetic acid (1.21 ml) and methanol (25 ml) was heated under reflux with stirring for 90 minutes. Water was added to the reaction mixture, and the precipitated crystals were collected by filtration to give N-(2-methoxycarbonylethyl)-2-[4-(N-methyl-N-benzylamidino)benzoylimino]-3,4-dimethyl-3H-thiazoline-5-carboxamide hydroiodide (Compound 34) (0.36 g).
m.p. 205 - 206°C

### Example 14

A mixture of Compound 34 (0.3 g), water (0.9 ml) and 47% aqueous hydrobromic acid solution (0.9 ml) was stirred at 80°C for 1.5 hours. The reaction mixture was ice-cooled, and the precipitated crystals were collected by filtration to give N-(2-carboxyethyl)-2-[4-(N-methyl-N-benzylamidino)benzoylimino]-3,4-dimethyl-3H-thiazoline-5-carboxamide hydrobromide (Compound 35) (0.202 g).
m.p. 259.5 - 260°C

### Example 15

A mixture of Compound 3 (1.0 g), dimethylamine acetate (1.26 g) and methanol (25 ml) was heated under reflux with stirring for 90 minutes. The reaction mixture was evaporated under reduced pressure, and the precipitated crystals were washed with methanol and acetone to give N-(2-methoxycarbonylethyl)-2-[4-(N,N-dimethylamidino)benzoylimino]-3,4-dimethyl-3H-thiazoline-5-carboxamide hydroiodide (Compound 36) (0.42 g).
m.p. 254 - 256°C

### Example 16

Following the procedure similar to that of Example 14 using Compound 36, there was obtained N-(2-carboxyethyl)-2-[4-(N,N-dimethylamidino)benzoylimino]-3,4-dimethyl-3H-thiazoline-5-carboxamide hydrobromide (Compound 37).
m.p. 275 - 275.5°C

### Example 17

Methyl chlorocarbonate (0.18 ml) was added dropwise to a mixture of Compound 4 (1.0 g), triethylamine (0.66 ml), water (10 ml) and tetrahydrofuran (10 ml), followed by stirring at room temperature for 2 hours. The reaction mixture was concentrated under reduced pressure, and the precipitated crystals were collected by filtration to give N-(2-methoxycarbonylethyl)-2-[4-(N-methoxycarbonylamidino)benzoylimino]-3,4-dimethyl-3H-thiazoline-5-carboxamide (Compound 38) (0.722 g).
m.p. 280 - 281°C

### Example 18

Triethylamine (0.9 ml) was added dropwise to a mixture of Compound 4 (1.48 g), di-tert-butyldicarbonate (1.05 g), water (30 ml) and tetrahydrofuran (30 ml) under ice-cooling, followed by stirring at room temperature for 2 hours. The reaction mixture was concentrated under reduced pressure, and the precipitated crystals were collected by filtration to give N-(2-methoxycarbonylethyl)-2-[4-(N-tert-butoxycarbonylamidino)-benzoylimino]-3,4-dimethyl-3H-thiazoline-5-carboxamide (Compound 39) (1.47 g).
m.p. 202 - 203°C

### Example 19

A mixture of Compound 39 (0.906 g), 10% sodium hydroxide solution (0.88 ml) and methanol (10 ml) was stirred at room temperature for an hour. The reaction mixture was concentrated under reduced pressure, dioxane was added thereto, and the precipitated crystals were collected by filtration to give sodium N-(2-carboxyethyl)-2-[4-(N-tert-butoxycarbonylamidino)benzoylimino]-3,4-dimethyl-3H-thiazoline-5-carboxamide (Compound 40) (0.58 g).
¹H-NMR (DMSO-d₆) δ (ppm):
   1.45 (9H, s), 2.09 (2H, t, J=6Hz), 2.62 (3H, s), 3.43 (2H, q, J=6Hz), 3.83 (3H, s), 8.03 (2H, d, J=8Hz), 8.26 (2H, d, J=8Hz), 9.00 (1H, t, J=6Hz), 9.12 (2H, brs)

### Example 20

β-Alanine benzyl ester p-toluenesulfonate (8.68 g), 1-hydroxybenzotriazole monohydrate (6.88 g) and 1-ethyl-3-[3-(dimethylamino)propyl]-carbodiimide hydrochloride (4.73 g) were successively added to a suspension of the compound obtained in Example 1(2) (7.26 g) in DMF (150 ml) with stirring, followed by stirring at room temperature for 14 hours. The reaction mixture was taken up in water, and the precipitated crystals were collected by filtration and recrystallized from a mixture of methylene chloride and hexane to give N-(2-benzyloxycarbonylethyl)-2-(4-cyanobenzoylimino)-3,4-dimetyl-3H-thiazoline-5-carboxamide (Compound 41) (9.37 g).
¹H-NMR (DMSO-d₆) δ (ppm):
   2.68 (2H, t, J=7Hz), 2.70 (3H, s), 3.69 (2H, q, J=7Hz), 3.85 (3H, s), 5.16 (2H, s), 6.40 (1H, t, J=7Hz), 7.3-7.4 (5H, m), 7.75 (2H, m), 8.42 (2H, m)

### Example 21

Following the reaction procedure similar to that of each of Examples 2 to 4 using Compound 41, there were obtained the following compounds.

N-(2-Benzyloxycarbonylethyl)-2-(4-thiocarbamoylbenzoylimino)-3,4-dimethyl-3H-thiazoline-5-carboxamide (Compound 42)
m.p. 179 - 180.5°C

N-(2-Benzyloxycarbonylethyl)-2-[4-(methylthioimidoyl)-benzoylimino]-3,4-dimethyl-3H-thiazoline-5-carboxamide hydroiodide (Compound 43)
m.p. 174.5 - 175°C

N-(2-Benzyloxycarbonylethyl)-2-(4-amidinobenzoylimino)-3,4-dimethyl-3H-thiazoline-5-carboxamide acetate (Compound 44)
m.p. 221 - 221.5°C

### Example 22

A mixture of Compound 18 (5.0 g), 10% sodium hydroxide solution (21 ml), methylene chloride (50 ml) and methanol (100 ml) was stirred at room temperature for 30 minutes. The reaction mixture was concentrated under reduced pressure and poured into 3% aqueous hydrochloric acid solution, and the precipitated crystals were collected by filtration to give N-(2-carboxyethyl)-2-(4-cyanobenzoylimino)-3-butyl-4-methyl-3H-thiazoline-5-carboxamide (Compound 45) (4.34 g).
m.p. 201 - 206°C

### Example 23

1-Ethyl-3-[3-(dimethylamino)propyl]-carbodiimide hydrochloride (2.13 g) was added to a mixture of Compound 45 (4.14 g), 2-hydroxymethylpyridine (1.18 g), 4-dimethylaminopyridine (0.24 g), 1-hydroxybenzotriazole monohydrate (3.06 g) and DMF (50 ml), followed by stirring at room temperature for 16 hours. The reaction mixture was poured into water and extracted with ethyl acetate. The ethyl acetate layer was washed with a saturated aqueous sodium chloride solution, dried over anhydrous magnesium sulfate, and evaporated under reduced pressure. The residue was chromatographed on silica gel column with ethyl acetate to give N-[2-(pyridin-2-ylmethoxycarbonyl)ethyl]-2-(4-cyanobenzoylimino)-3-butyl-4-methyl-3H-thiazoline-5-carboxamide (Compound 46) (2.98 g).
m.p. 127 - 129°C

### Example 24

Following the reaction procedure similar to that of each of Examples 2 to 4 using Compound 46, there were obtained the following compounds.

N-[2-(Pyridin-2-ylmethoxycarbonyl)ethyl]-2-(4-thiocarbamoylbenzoylimino)-3-butyl-4-methyl-3H-thiazoline-5-carboxamide (Compound 47)
m.p. 134 - 135°C

N-[2-(Pyridin-2-ylmethoxycarbonyl)ethyl]-2-[4-(methylthioimidoyl)-benzoylimino]-3-butyl-4-methyl-3H-thiazoline-5-carboxamide hydroiodide-acetone solvate (Compound 48)
m.p. 150.5 - 152°C

N-[2-(Pyridin-2-ylmethoxycarbonyl)ethyl]-2-(4-amidinobenzoylimino)-3-butyl-4-methyl-3H-thiazoline-5-carboxamide acetate (Compound 49)
m.p. 202 - 204°C

### Example 25

3-Aminopropanol (0.13 ml), 1-hydroxybenzotriazole monohydrate (0.473 g) and 1-ethyl-3-[3-(dimethylamino)propyl]-carbodiimide hydrochloride (0.326 g) were successively added to a suspension of the compound obtained in 1(2) (0.5 g) in DMF (10 ml), followed by stirring at room temperature for 14 hours. The reaction mixture was taken in water, and the precipitated crystals were collected by filtration, and recrystallized from a mixture of ethyl acetate and hexane to give N-(3-hydroxypropyl)-2-(4-cyanobenzoylimino)-3,4-dimethyl-3H-thiazoline-5-carboxamide (Compound 50) (0.43 g).
m.p. 210 - 212.5°C

### Example 26

Following the reaction procedure similar to that of each of Examples 2 to 4 using Compound 50, there were obtained the following compounds.

N-(3-Hydroxypropyl)-2-(4-thiocarbamoylbenzoylimino)-3,4-dimethyl-3H-thiazoline-5-carboxamide (Compound 51)
m.p. 226.5 - 227.5°C

N-(3-Hydroxypropyl)-2-[4-(methylthioimidoyl)-benzoylimino]-3,4-dimethyl-3H-thiazoline-5-carboxamide hydroiodide (Compound 52)
m.p. 206 - 207.5°C

N-(3-Hydroxypropyl)-2-(4-amidinobenzoylimino)-3,4-dimethyl-3H-thiazoline-5-carboxamide acetate (Compound 53)
m.p. 230 - 230.5°C

### Example 27

Glycine methyl ester hydrochloride (1.07 g), 1-hydroxybenzotriazole monohydrate (2.37 g) and 1-ethyl-3-[3-(dimethylamino)propyl]-carbodiimide hydrochloride (1.63 g) were successively added to a suspension of the compound obtained in Example 1(2) (2.5 g) in DMF (45 ml) with stirring, followed by stirring at room temperature for 14 hours. The reaction mixture was taken up in water, and the precipitated crystals were collected by filtration and recrystallized from a mixture of methylene chloride and hexane to give N-(methoxycarbonylmethyl)-2-(4-cyanobenzoylimino)-3,4-dimethyl-3H-thiazoline-5-carboxamide (Compound 54) (2.25 g).
m.p. 238 - 238.5°C

### Example 28

Following the reaction procedure similar to that of each of Examples 2 to 4 using Compound 54, there were obtained the following compounds.

N-(Methoxycarbonylmethyl)-2-(4-thiocarbamoylbenzoylimino)-3,4-dimethyl-3H-thiazoline-5-carboxamide (Compound 55)
m.p. 235 - 235.5°C

N-(Methoxycarbonylmethyl)-2-[4-(methylthioimidoyl)-benzoylimino]-3,4-dimethyl-3H-thiazoline-5-carboxamide hydroiodide (Compound 56)
m.p. 216.5 - 217°C

N-(Methoxycarbonylmethyl)-2-(4-amidinobenzoylimino)-3,4-dimethyl-3H-thiazoline-5-carboxamide acetate (Compound 57)
m.p. 236.5 - 237°C

### Example 29

A mixture of Compound 57 (0.15 g), 10% aqueous sodium hydroxide solution (0.3 ml) and methanol (5 ml) was stirred at 50°C for 3 hours. 3% Hydrochloric acid was added to the reaction mixture, and the precipitated crystals were collected by filtration to give N-(carboxymethyl)-2-(4-amidinobenzoylimino)-3,4-dimethyl-3H-thiazoline-5-carboxamide hydrochloride (Compound 58) (0.072 g).
m.p. 289 - 289.5°C

### Example 30

Methyl 4-aminobutyrate hydrochloride (1.57 g), 1-hydroxybenzotriazole monohydrate (2.84 g) and 1-ethyl-3-[3-(dimethylamino)propyl]-carbodiimide hydrochloride (1.96 g) were successively added to a suspension of the compound obtained in Example 1(2) (3.0 g) in DMF (55 ml) with stirring, followed by stirring at room temperature for 14 hours. The reaction mixture was taken up in water, and the precipitated crystals were collected by filtration and recrystallized from a mixture of methylene chloride and hexane to give N-(4-methoxycarbonylpropyl)-2-(4-cyanobenzoylimino)-3,4-dimethyl-3H-thiazoline-5-carboxamide (Compound 59) (3.27 g).
m.p. 182 - 184.5°C

### Example 31

Following the reaction procedure similar to that of each of Examples 2 to 4 using Compound 59, there were obtained the following compounds.

N-(4-Methoxycarbonylpropyl)-2-(4-thiocarbamoylbenzoylimino)-3,4-dimethyl-3H-thiazoline-5-carboxamide (Compound 60)
¹H-NMR (DMSO-d₆) δ (ppm):
   1.76 (2H, m), 2.36 (2H, t, J=7Hz), 2.60 (3H, s), 3.23 (2H, q, J=7Hz), 3.60 (3H, s), 3.82 (3H, s), 7.95 (2H, d, J=8Hz), 8.22 (2H, d, J=8Hz), 9.62 (1H, s), 9.98 (1H, s)

N-(4-Methoxycarbonypropyl)-2-[4-(methylthioimidoyl)-benzoylimino]-3,4-dimethyl-3H-thiazoline-5-carboxamide hydroiodide (Compound 61)
m.p. 188 - 189°C

N-(4-Methoxycarbonylpropyl)-2-(4-amidinobenzoylimino)-3,4-dimethyl-3H-thiazoline-5-carboxamide hydrobromide (Compound 62)
m.p. 234.5 - 237°C

### Example 32

(1) Ethyl 2-(4-cyanobenzoylamino)-4-methyl-thiazole-5-carboxylate (5.0 g) was added to a suspension of 60% sodium hydride in oil (0.76 g) in DMF (60 ml) under ice-cooling, followed by stirring at room temperature for an hour. A solution of 1-chloromethylnaphthalene (3.37 g) in DMF (5 ml) was added dropwise to the reaction mixture, followed by further stirring at room temperature for 14 hours. The reaction mixture was taken up in 3% hydrochloric acid, the precipitated crystals were collected by filtration and chromatographed on silica gel column with methylene chloride, and the resulting crude crystals were recrystallized from a mixture of methylene chloride and methanol to give ethyl 2-(4-cyanobenzoylimino)-3-(1-naphthylmethyl)-4-methyl-3H-thiazoline-5-carboxylate (1.86 g).
   m.p. 229 - 229.5°C
(2) Following the reaction procedures similar to those of Example 1(2) and 1(3) using ethyl 2-(4-cyanobenzoylimino)-3-(1-naphthylmethyl)-4-methylthiazoline-5-carboxylate, there was obtained N-(2-methoxycarbonylethyl)-2-(4-cyanobenzoylimino)-3-(1-naphthylmethyl)-4-methyl-3H-thiazoline-5-carboxamide (Compound 63).
   m.p. 230 - 231°C

### Example 33

Following the reaction procedure similar to that of each of Examples 2 and 3 using compound 63, there were obtained the following compounds.

N-(4-Methoxycarbonylethyl)-2-(4-thiocarbamoylbenzoylimino)-3-(1-naphthylmethyl)-4-methyl-3H-thiazoline-5-carboxamide (Compound 64)
m.p. 217 - 218°C

N-(4-Methoxycarbonylethyl)-2-[4-(methylthioimidoyl)-benzoylimino]-3-(1-naphthylmethyl)-4-methyl-3H-thiazoline-5-carboxamide hydroiodide (Compound 65)
m.p. 166 - 169°C

### Example 34

(1) Ethyl 2-(4-cyanobenzoylamino)-4-methylthiazole-5-carboxylate (5.0 g) was added to a suspension of 60% sodium hydride in oil (0.76 g) in DMF (60 ml) under ice-cooling, followed by stirring at room temperature for an hour. 1-Iodohexane (8.46 ml) was added dropwise to the reaction mixture, followed by further stirring at room temperature for 14 hours. The reaction mixture was taken up in 3% hydrochloric acid and extracted with methylene chloride. The methylene chloride layer was evaporated under reduced pressure and crystallized from hexane to give ethyl 2-(4-cyanobenzoylimino)-3-hexyl-4-methyl-3H-thiazoline-5-carboxylate (3.86 g).
   m.p. 125 - 126°C
(2) Following the reaction procedures similar to those of Example 1(2) and 1(3) using ethyl 2-(4-cyanobenzoylimino)-3-hexyl-4-methyl-3H-thiazoline-5-carboxylate, there was obtained N-(2-methoxycarbonylethyl)-2-(4-cyanobenzoylimino)-3-hexyl-4-methyl-3H-thiazoline-5-carboxamide (Compound 66).
   m.p. 100 - 104°C

### Example 35

Following the reaction procedures similar to that of each of Examples 2 to 4 using Compound 66, there were obtained the following compounds.

N-(2-Mehoxycarbonylethyl)-2-(4-thiocarbamoylbenzoylimino)-3-hexyl-4-methyl-3H-thiazoline-5-carboxamide (Compound 67)
m.p. 176.5 - 178°C

N-(2-Methoxycarbonylethyl)-2-[4-(methylthioimidoyl)-benzoylimino]-3-hexyl-4-methyl-3H-thiazoline-5-carboxamide hydroiodide (Compound 68)
m.p. 192.5 - 194°C

N-(2-Methoxycarbonylethyl)-2-(4-amidinobenzoylimino)-3-hexyl-4-methyl-3H-thiazoline-5-carboxamide acetate (Compound 69)
m.p. 190.5 - 192°C

N-(2-Carboxyethyl)-2-(4-amidinobenzoylimino)-3-hexyl-4-methyl-3H-thiazoline-5-carboxamide hydrobromide (Compound 70)
m.p. 186.5 - 190°C

### Example 36

(1) A mixture of methyl 4-formylbenzoate (11.5 g), sulfur (2.28 g) and morpholine (140 ml) was heated under reflux for 20 minutes. The reaction mixture was allowed to stand for cooling, the insolubles were filtered off, and the filtrate was poured into 3% hydrochloric acid and extracted with ethyl acetate. The ethyl acetate layer was washed with 5% aqueous sodium bicarbonate solution and a saturated aqueous sodium chloride solution, dried over anhydrous magnesium sulfate, evaporated under reduced pressure, and recrystallized from an aqueous methanol to give methyl 4-(morpholinothiocarbonyl)benzoate (15.7 g).
   m.p. 123.5 - 125°C
(2) A mixture of the compound obtained in (1) (6.1 g), 10% sodium hydroxide solution (18 ml) and methanol (100 ml) was stirred at room temperature for 2 hours. The reaction mixture was concentrated under reduced pressure and poured into 3% hydrochloric acid, and the precipitated crystals were collected by filtration to give 4-(morpholinothiocarbonyl)benzoic acid (5.29 g).
   m.p. 227 - 230°C
(3) Triethylamine (3.1 ml) was added dropwise to a mixture of the compound obtained in (2) (5.0 g), ethyl 2-amino-4-methylthiazole-5-carboxylate hydrochloride (4.43 g), 1-hydroxybenzotriazole monohydrate (6.09 g), 1-ethyl-3-[3-(dimethylamino)propyl]-carbodiimide hydrochloride (4.22 g) and DMF (100 ml), followed by stirring at room temperature for 14 hours and then at 60°C for 3 hours. The reaction mixture was taken up in water, and the precipitated crystals were collected by filtration and recrystallized from a mixture of ethyl acetate and hexane to give ethyl 2-[4-(morpholinothiocarbonyl)benzoylamino]-4-methylthiazole-5-carboxylate (8.34 g).
   m.p. 265°C (decomposition)
(4) Following the reaction procedure similar to that of Example 1 using the compound obtained in (3), there were obtained the following compounds.

Ethyl 2-[4-(morpholinothiocarbonyl)benzoylimino]-3,4-dimethyl-3H-thiazoline-5-carboxylate
m.p. 203 - 206°C

N-(2-Methoxycarbonylethyl)-2-[4-(morpholinothiocarbonyl)benzoylimino]-3,4-dimethyl-3H-thiazoline-5-carboxamide (Compound 71)
m.p. 227.5 - 231°C

### Example 37

(1) Following the reaction procedure similar to that of Example 3 using Compound 71, there was obtained α-methylthio-4-{[5-(2-(methoxycarbonyl)ethylaminocarbonyl]-3,4-dimethyl-3H-thiazoline-2-dene]aminocarbonyl}benzylidene morpholinium iodide.
   m.p. 202.5 - 205°C
(2) A mixture of α-methylthio-4-{[5-[2-(methoxycarbonyl)ethylaminocarbonyl]-3,4-dimethyl-3H-thiazoline-2-dene]aminocarbonyl}benzylidene morpholinium iodide (3.02 g), 1,2-diaminoethane (0.3 g) and methanol (100 ml) was stirred at room temperature for 2 hours. The reaction mixture was concentrated under reduced pressure, and the precipitated crystals were collected by filtration to give N-(2-methoxycarbonylethyl)-2-[4-(imidazolin-2-yl)benzoylimino]-3,4-dimethyl-3H-thiazoline-5-carboxamide acetate (Compound 72).
   ¹H-NMR (DMSO-d₆) δ (ppm):
      2.58 (2H, t, J=6Hz), 2.60 (3H, s), 3.46 (2H, q, J=6Hz), 3.61 (4H, m), 3.85 (6H, s), 7.98 (2H, m), 8.32 (1H, t, J=6Hz), 8.34 (2H, m)

### Example 38

Following the reaction procedure similar to that of Example 29 using Compound 72, there was obtained N-(2-carboxyethyl)-2-[4-(imidazolin-2-yl)benzoylimino]-3,4-dimethyl-3H-thiazoline-5-carboxamide hydrochloride (Compound 73).
¹H-NMR (DMSO-d₆) δ (ppm):
   2.27 (3H, s), 2.63 (2H, t, J=6Hz), 3.50 (2H, t, J=6Hz), 3.58 (3H, s), 4.08 (4H, brs), 7.69 (2H, m), 8.06 (2H, m)

### Example 39

Following the reaction procedure similar to that of each of Examples 4 and 5 using Compound 3 and the corresponding materials, there were obtained the following compounds shown in Table 4.

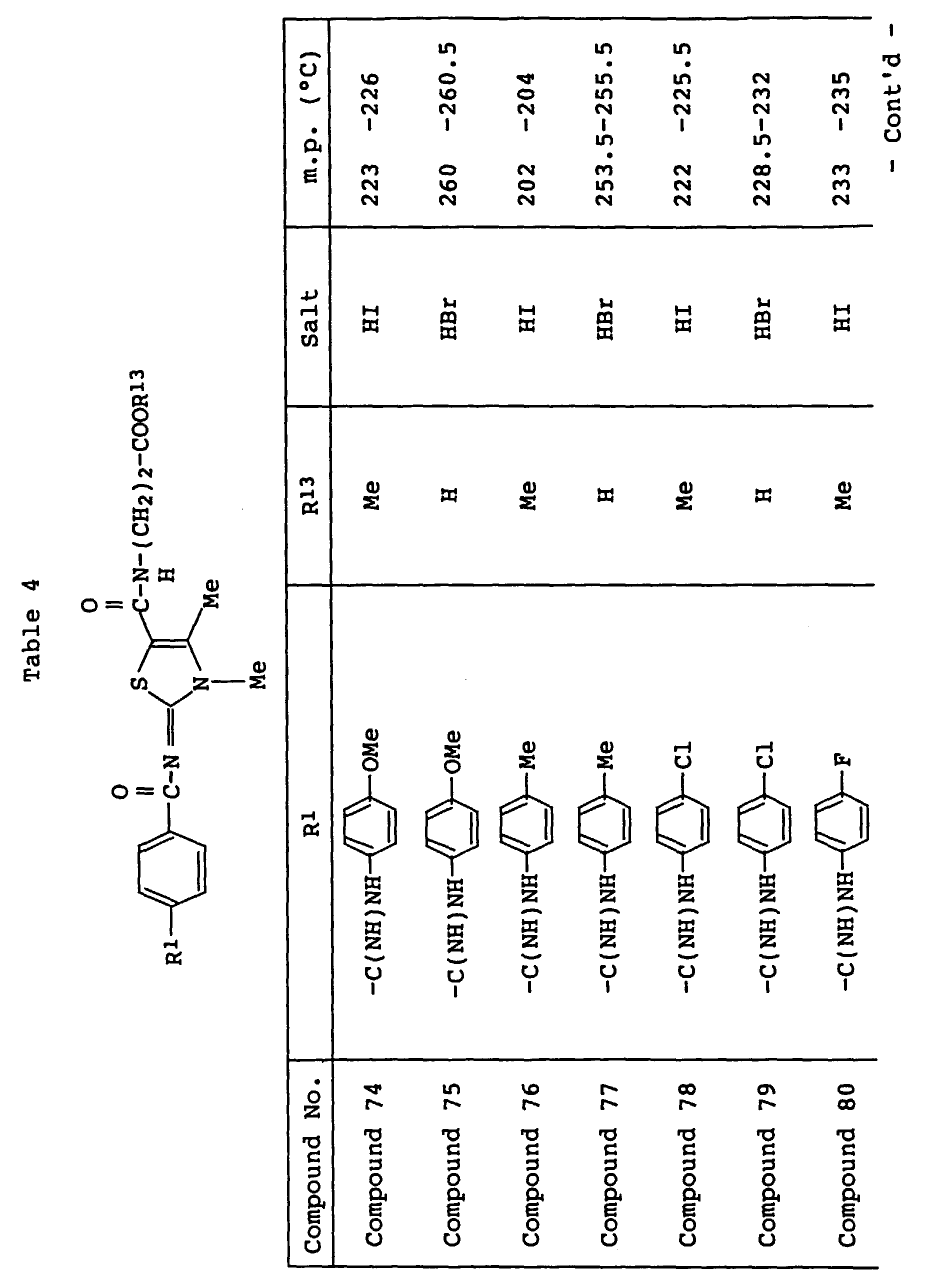

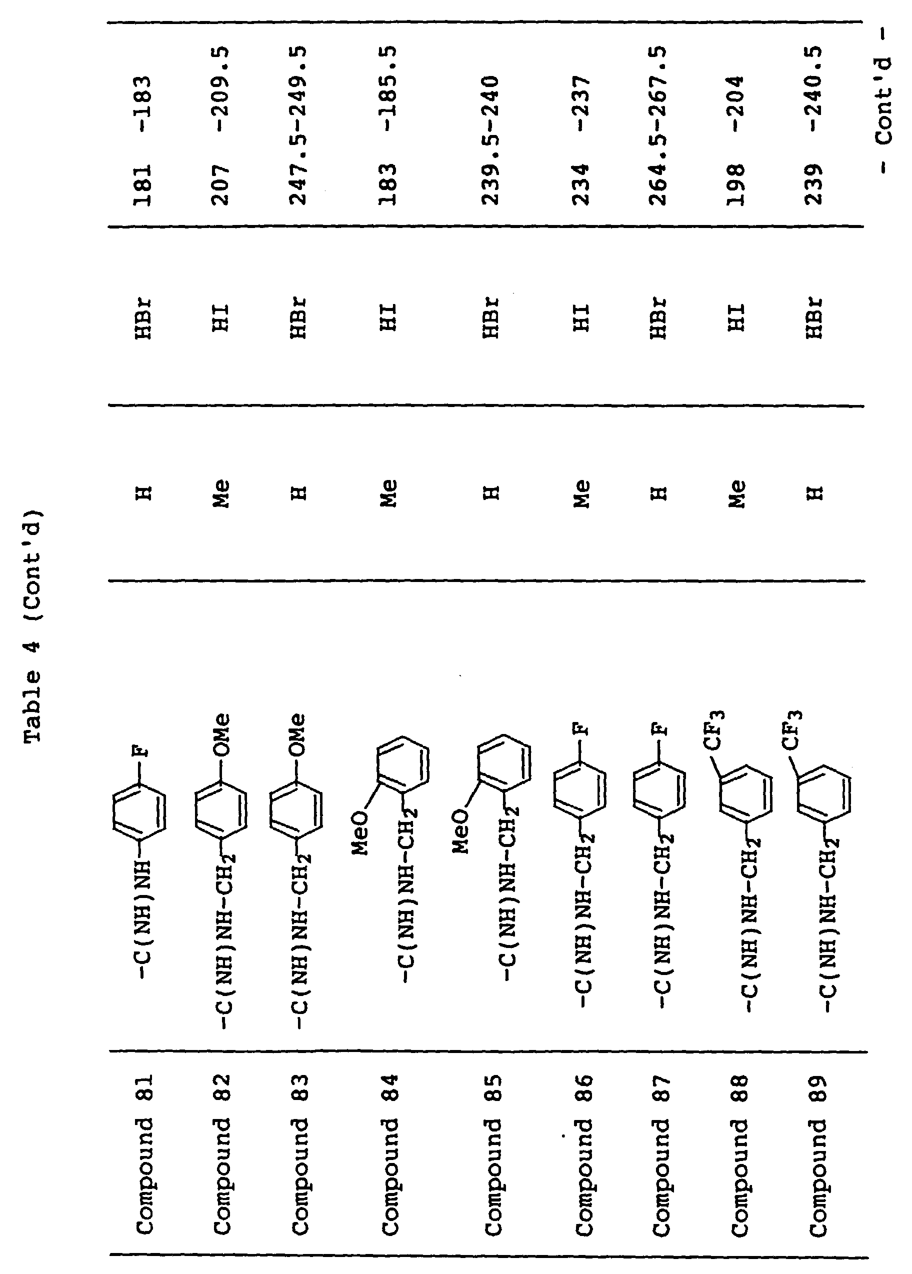

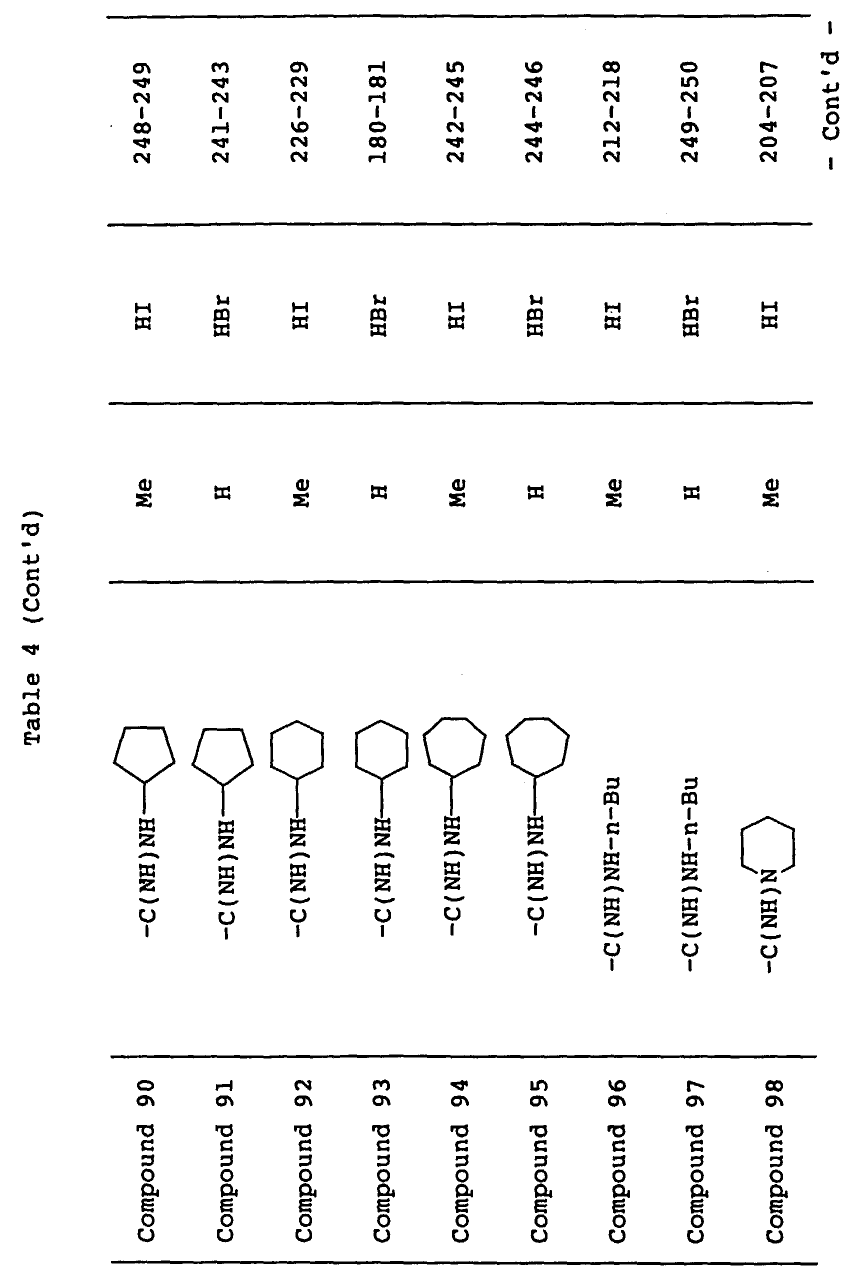

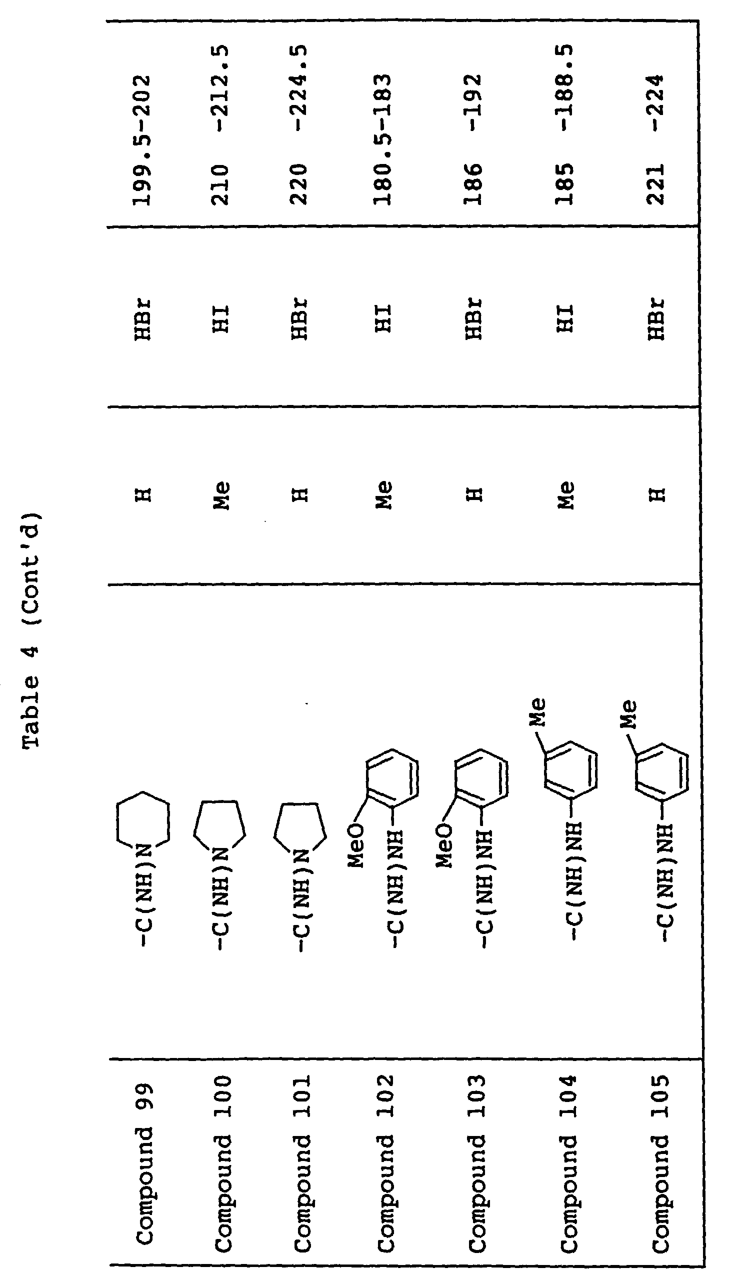

### Example 40

Following the reaction procedure similar to that of each of Examples 4 and 5 using the compound obtained in Example 37(1), there were obtained the following compounds.

N-(2-Methoxycarbonylethyl)-2-[4-(morpholinoimidoyl)benzoylimino]-3,4-dimethyl-3H-thiazoline-5-carboxamide hydroiodide (Compound 106)
m.p. 247 - 248°C

N-(2-Carboxyethyl)-2-[4-(morpholinoimidoyl)-benzoylimino]-3,4-dimethyp-3H-thiazoline-5-carboxamide hydrobromide (Compound 107)
m.p. 258.5 - 260°C

## Claims (Claims for the following Contracting State(s): AT, BE, CH, LI, DE, DK, FR, GB, IE, IT, LU, MC, NL, PT, SE)

1. A thiazoline derivative represented by the formula: 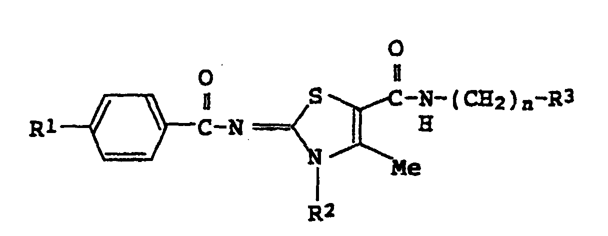 wherein R¹ is a cyano group, a carbamoyl group, a thiocarbamoyl group, a morpholinothiocarbonyl group, an alkylthioimidoyl group having 2 to 7 carbon atoms, a group represented by the formula: 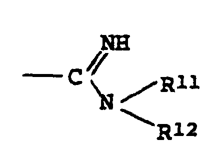 wherein R¹¹ and R¹² are the same or different and are each a hydrogen atom, an alkyl group having 1 to 6 carbon atoms, an alkoxycarbonyl group having 2 to 7 carbon atoms, a cycloalkyl group having 4 to 8 carbon atoms, a phenyl group, a phenyl group substituted by an alkyl group having 1 to 4 carbon atoms, an alkoxy group having 1 to 4 carbon atoms or a halogen atom, an aralkyl group being an alkyl group having 1 to 3 carbon atoms substituted by a phenyl or a naphthyl group at the end thereof, or the aralkyl group substituted by an alkyl group having 1 to 4 carbon atoms, an alkoxy group having 1 to 4 carbon atoms, a trifluoromethyl group or a halogen atom or R¹¹ and R¹² together with the adjacent nitrogen atom form a univalent group of a 4- to 7-membered heterocyclic ring having at least one nitrogen atom on the ring which may have a substituent selected from alkyl, phenyl or benzyl at the 4-position or an imidazolin-2-yl group, n is an integer of 1 to 3, R² is an alkyl group having 1 to 10 carbon atoms or an aralkyl group being an alkyl group having 1 to 3 carbon atoms substituted by a phenyl or a naphthyl group at the end thereof, and R³ is a carboxyl group, a hydroxyl group, an alkoxycarbonyl group having 2 to 7 carbon atoms, a benzyloxycarbonyl group or a pyridylmethyloxycarbonyl group or a salt thereof.

2. Use of a thiazoline derivative as claimed in claim 1 for the preparation for preventive or therapeutic agents for ischemic diseases and atherosclerosis diseases, or metastasis inhibitory agents for malignant tumors.

## Claims (Claims for the following Contracting State(s): GR, ES)

1. A thiazoline derivative represented by the formula: 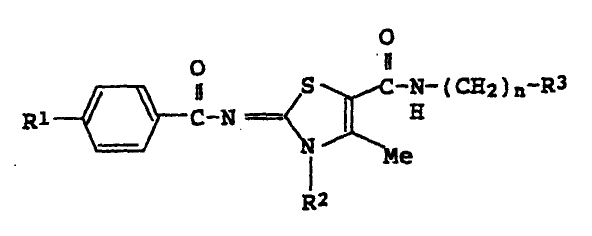 wherein R¹ is a cyano group, a carbamoyl group, a thiocarbamoyl group, a morpholinothiocarbonyl group, an alkylthioimidoyl group having 2 to 7 carbon atoms, a group represented by the formula: 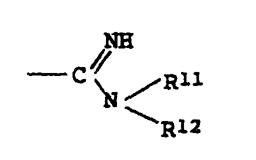 wherein R¹¹ and R¹² are the same or different and are each a hydrogen atom, an alkyl group having 1 to 6 carbon atoms, an alkoxycarbonyl group having 2 to 7 carbon atoms, a cycloalkyl group having 4 to 8 carbon atoms, a phenyl group, a phenyl group substituted by an alkyl group having 1 to 4 carbon atoms, an alkoxy group having 1 to 4 carbon atoms or a halogen atom, an aralkyl group being an alkyl group having 1 to 3 carbon atoms substituted by a phenyl or a naphthyl group at the end thereof, or the aralkyl group substituted by an alkyl group having 1 to 4 carbon atoms, an alkoxy group having 1 to 4 carbon atoms, a trifluoromethyl group or a halogen atom or R¹¹ and R¹² together with the adjacent nitrogen atom form a univalent group of a 4- to 7-membered heterocyclic ring having at least one nitrogen atom on the ring which may have a substituent selected from alkyl, phenyl or benzyl at the 4-position or an imidazolin-2-yl group, n is an integer of 1 to 3, R² is an alkyl group having 1 to 10 carbon atoms or an aralkyl group being an alkyl group having 1 to 3 carbon atoms substituted by a phenyl or a naphthyl group at the end thereof, and R³ is a carboxyl group, a hydroxyl group, an alkoxycarbonyl group having 2 to 7 carbon atoms, a benzyloxycarbonyl group or a pyridylmethyloxycarbonyl group or a salt thereof.

2. Use of a thiazoline derivative as claimed in claim 1 for the preparation for preventive or therapeutic agents for ischemic diseases and atherosclerosis diseases, or metastasis inhibitory agents for malignant tumors.

3. A process for the preparation of a thiazoline derivative as claimed in claim 1, comprising the following steps:
(i) reacting a compound represented by the formula (III) 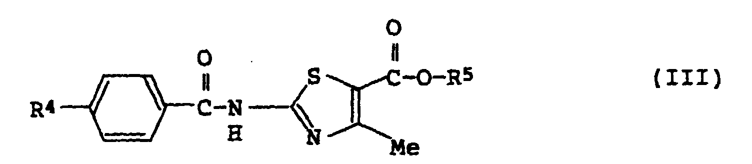 wherein R⁴ is a cyano group or a morpholinothiocarbonyl group, and R⁵ is a lower alkyl group, a benzyl group or a 2-cyanoethyl group with a halide represented by the formula:
R²-X
wherein R² is as defined in claim 1, and X is a halogen atom in the presence of a base to give a compound represented by the formula IV 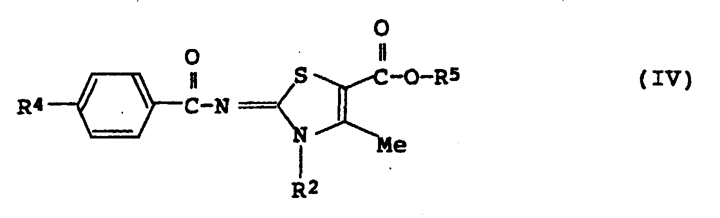 wherein R², R⁴ and R⁵ are as defined above,
or by reacting a compound represented by the formula (III) as defined above with an alkylating agent represented by the formula:
R⁶₂-SO₄
wherein R⁶ is R² other than an aralkyl group, or a sulfonate represented by the formula:
R⁷SO₃R⁶
wherein R⁷ is an alkyl group or an aryl group, and R⁶ is as defined above to obtain a compound of formula IV 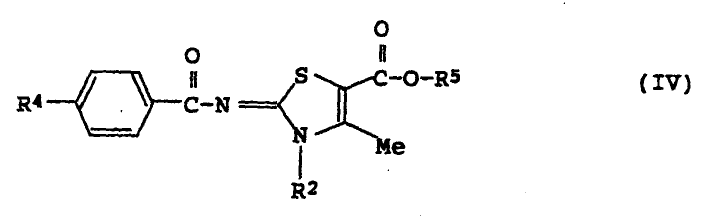 wherein R² is other than an aralkyl group;
(ii) hydrolyzing the ester moiety of the compound of formula (IV) to obtain a compound represented by the formula: 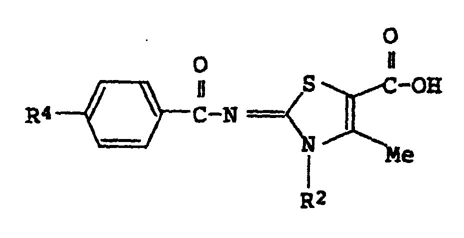 wherein R² and R⁴ are as defined above or a salt thereof;
(iii) reacting said compound obtained by step (ii) with an amine represented by the formula:
H₂N-(CH₂)ₙ-R³
wherein n and R³ are as defined in claim 1 to obtain a thiazoline derivative as claimed in claim 1, wherein R¹ is a cyano group or a morpholinothiocarbonyl group.

4. A process for the preparation of a thiazoline derivative as claimed in claim 3, wherein R¹ is a thiocarbamoyl group, which comprises subjecting a thiazoline derivative represented by the formula: 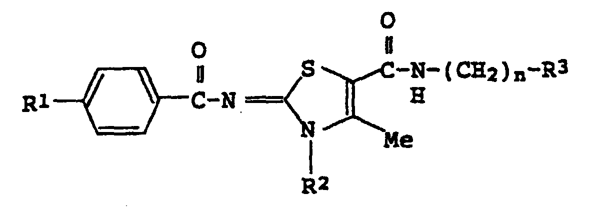 wherein R¹ is a cyano group, and R², R³ and n are as defined in claim 1 to a reaction with hydrogen sulfide using a base as a catalyst or to a reaction with
NaBH₂S₃.

5. A process for the preparation of a thiazoline derivative as claimed in claim 3, wherein R¹ is a carbamoyl group, which comprises reacting a thiazoline derivative represented by the formula: 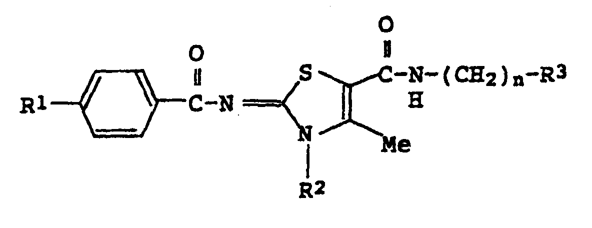 wherein R¹ is a cyano group, and R², R³ and n are as defined in claim 1 with hydrogen peroxide in the presence of a base.

6. A process for the preparation of a thiazoline derivative as claimed in claim 3, wherein R¹ is an alkylthioimidoyl group having 2 to 7 carbon atoms, which comprises treating a thiazoline derivative represented by the formula: 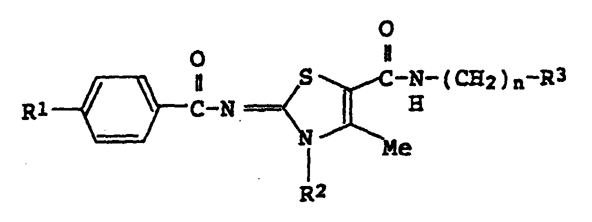 wherein R¹ is a thiocarbamoyl group, and R², R³ and n are as defined in claim 1 with an alkyl halide represented by the formula:
R⁸-X
wherein R⁸ is a lower alkyl group, and X is a halogen atom.

7. A process for the preparation of a thiazoline derivative as claimed in claim 3, wherein R¹ is a group of formula (II) 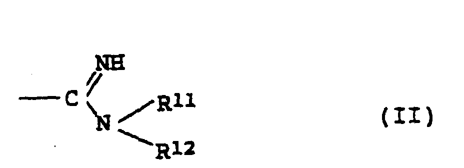 wherein R¹¹ or R¹² is other than an alkoxycarbonyl group having 2 to 7 carbon atoms or a imidazolin-2-yl group, which comprises reacting a thiazoline derivative represented by the formula: 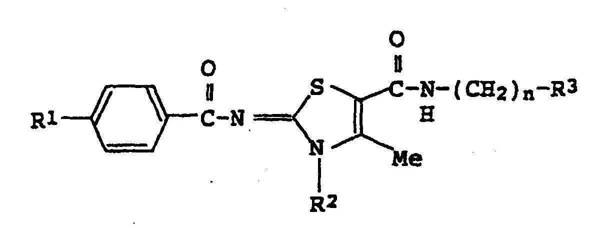 wherein R¹ is an alkylthioimidoyl group having 2 to 7 carbon atoms with ammonia, a monoalkylamine having 1 to 6 carbon atoms, a dialkylamine having 1 to 6 carbon atoms, a monocycloalkylamine having 4 to 8 carbon atoms, aniline, a substituted aniline, an aralkylamine, a substituted aralkylamine, a heterocyclic compound having one or more secondary nitrogen atoms on the ring, 1,2-diaminoethane or a salt thereof.

8. A process for the preparation of a thiazoline derivative as claimed in claim 3, wherein R¹ is a group of formula (II), R¹¹ and/or R¹² is an alkoxycarbonyl group having 2 to 7 carbon atoms, which comprises reacting a compound obtained by the process according to claim 7,
wherein R¹ is a group of the formula (II), and R¹¹ and R¹² are other than an alkoxycarbonyl group having 2 to 7 carbon atoms with a compound represented by the formula:
R⁹-O(C=O)-X
or
R⁹-O(C=O)-O-(C=O)O-R⁹
wherein R⁹ is a lower alkyl group, and X is a halogen atom in the presence of a base.

9. A process for the preparation of a thiazoline derivative as claimed in claim 3, wherein R³ is a carboxyl group or a salt thereof, which comprises an ester hydrolysis of the compound of formula (I), wherein R³ is other than a carboxyl group or a hydroxyl group by alkali treatment or mineral acid treatment.

10. A process for the preparation of a thiazoline derivative as claimed in claim 3, wherein R³ is an alkoxycarbonyl group having 2 to 7 carbon atoms, a benzyloxycarbonyl group or a pyridylmethyloxycarbonyl group, which comprises reacting a compound of the formula: 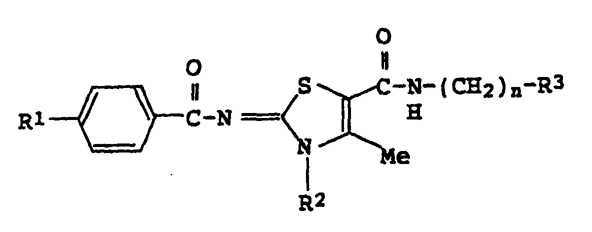 as defined in claim 1, wherein R³ is a carboxyl group or a salt thereof with a compound represesented by the formula:
R¹⁰-OH
or wherein R¹⁰ is a lower alkyl group, a benzyl group or a pyridylmethyl group, and X is a halogen atom under esterification conditions.

11. A process for the preparation of a thiazoline derivative as claimed in claim 3, wherein R³ is an alkoxycarbonyl group having 2 to 7 carbon atoms, a benzyloxycarbonyl group or a pyridylmethyloxycarbonyl group, which comprises esterinter-changing.

12. A process for the preparation of a thiazoline derivative as claimed in claim 3, wherein R¹ is an imidazolin-2-yl group by reacting a compound represented by the formula: 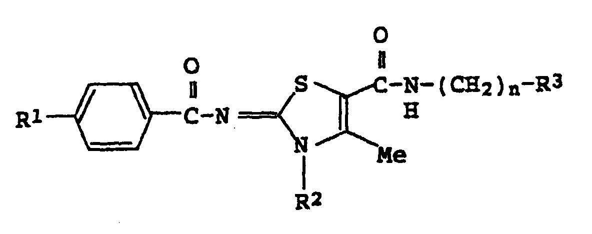 wherein R¹ is a morpholinothiocarbonyl group, and R², R³ and n are as defined in claim 1 with a lower alkyl halide represented by the formula:
R⁶-X
wherein R⁸ is a lower alkyl group, and X is a halogen atom and reacting the resulting compound with 1,2-diaminoethane or a salt thereof.

## Patentansprüche (Patentansprüche für folgende(n) Vertragsstaat(en): AT, BE, CH, LI, DE, DK, FR, GB, IE, IT, LU, MC, NL, PT, SE)

1. Thiazolinderivat, dargestellt durch die Formel: 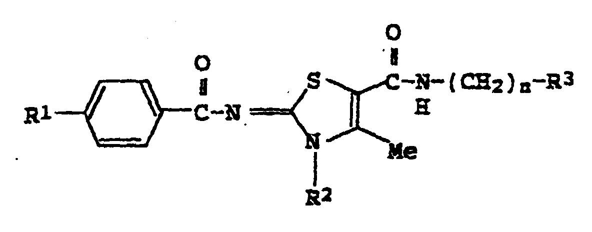 worin R¹ eine Cyanogruppe, eine Carbamoylgruppe, eine Thiocarbamoylgruppe, eine Morpholinothiocarbonylgruppe, eine Alkylthioimidoylgruppe mit 2 bis 7 Kohlenstoffatomen, eine Gruppe dargestellt durch die Formel: 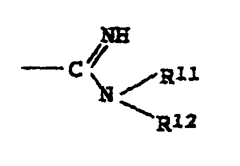 bedeutet, worin R¹¹ und R¹² gleich oder unterschiedlich sind und jeweils ein Wasserstoffatom, eine Alkylgruppe mit 1 bis 6 Kohlenstoffatomen, eine Alkoxycarbonylgruppe mit 2 bis 7 Kohlenstoffatomen, eine Cycloalkylgruppe mit 4 bis 8 Kohlenstoffatomen, eine Phenylgruppe, eine Phenylgruppe substituiert mit einer Alkylgruppe mit 1 bis 4 Kohlenstoffatomen, eine Alkoxygruppe mit 1 bis 4 Kohlenstoffatomen oder ein Halogenatom, eine Aralkylgruppe, welche eine Alkylgruppe mit 1 bis 3 Kohlenstoffatomen ist, die am Ende mit einer Phenyl- oder Naphthylgruppe substituiert ist, oder die Aralkylgruppe ist mit einer Alkylgruppe mit 1 bis 4 Kohlenstoffatomen substituiert, eine Alkoxygruppe mit 1 bis 4 Kohlenstoffatomen, eine Trifluormethylgruppe oder ein Halogenatom bedeuten, oder R¹¹ und R¹² bilden zusammen mit dem benachbarten Stickstoffatom eine univalente Gruppe eines 4- bis 7-gliedrigen heterocyclischen Rings mit mindestens einem Stickstoffatom im Ring, welcher einen Substituenten, ausgewählt aus Alkyl, Phenyl oder Benzyl an der 4-Position aufweisen kann, oder eine Imidazolin-2-yl-Gruppe, n eine ganze Zahl von 1 bis 3 ist, R² eine Alkylgruppe mit 1 bis 10 Kohlenstoffatomen, oder eine Aralkylgruppe, welche eine Alkylgruppe mit 1 bis 3 Kohlenstoffatomen, substituiert mit einer Phenyl- oder einer Naphthylgruppe am Ende davon, bedeutet, und R³ eine Carboxylgruppe, eine Hydroxylgruppe, eine Alkoxycarbonylgruppe mit 2 bis 7 Kohlenstoffatomen, eine Benzyloxycarbonylgruppe oder eine Pyridylmethyloxycarbonylgruppe oder ein Salz hiervon bedeutet.

2. Verwendung eines Thiazolinderivats nach Anspruch 1 für die Herstellung von präventiven oder therapeutischen Mitteln für ischämische Erkrankungen und atherosklerotische Erkrankungen, oder Metastasen-inhibierende Mittel für bösartige Tumore.

## Patentansprüche (Patentansprüche für folgende(n) Vertragsstaat(en): GR, ES)

1. Thiazolinderivat, dargestellt durch die Formel: 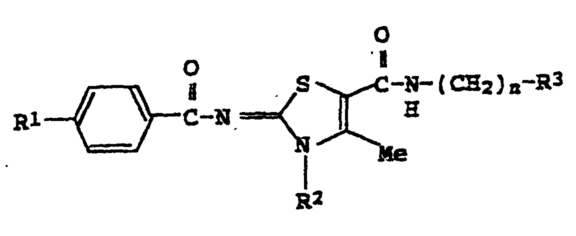 worin R¹ eine Cyanogruppe, eine Carbamoylgruppe, eine Thiocarbamoylgruppe, eine Morpholinothiocarbonylgruppe, eine Alkylthioimidoylgruppe mit 2 bis 7 Kohlenstoffatomen, eine Gruppe dargestellt durch die Formel: 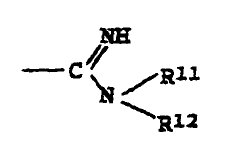 bedeutet, worin R¹¹ und R¹² gleich oder unterschiedlich sind und jeweils ein Wasserstoffatom, eine Alkylgruppe mit 1 bis 6 Kohlenstoffatomen, eine Alkoxycarbonylgruppe mit 2 bis 7 Kohlenstoffatomen, eine Cycloalkylgruppe mit 4 bis 8 Kohlenstoffatomen, eine Phenylgruppe, eine Phenylgruppe substituiert mit einer Alkylgruppe mit 1 bis 4 Kohlenstoffatomen, eine Alkoxygruppe mit 1 bis 4 Kohlenstoffatomen oder ein Halogenatom, eine Aralkylgruppe, welche eine Alkylgruppe mit 1 bis 3 Kohlenstoffatomen ist, die am Ende mit einer Phenyl- oder Naphthylgruppe substituiert ist, oder die Aralkylgruppe ist mit einer Alkylgruppe mit 1 bis 4 Kohlenstoffatomen substituiert, eine Alkoxygruppe mit 1 bis 4 Kohlenstoffatomen, eine Trifluormethylgruppe oder ein Halogenatom bedeuten, oder R¹¹ und R¹² bilden zusammen mit dem benachbarten Stickstoffatom eine univalente Gruppe eines 4- bis 7-gliedrigen heterocyclischen Rings mit mindestens einem Stickstoffatom im Ring, welcher einen Substituenten, ausgewählt aus Alkyl, Phenyl oder Benzyl an der 4-Position aufweisen kann, oder eine Imidazolin-2-yl-Gruppe, n eine ganze Zahl von 1 bis 3 ist, R² eine Alkylgruppe mit 1 bis 10 Kohlenstoffatomen, oder eine Aralkylgruppe, welche eine Alkylgruppe mit 1 bis 3 Kohlenstoffatomen, substituiert mit einer Phenyl- oder einer Naphthylgruppe am Ende davon, bedeutet, und R³ eine Carboxylgruppe, eine Hydroxylgruppe, eine Alkoxycarbonylgruppe mit 2 bis 7 Kohlenstoffatomen, eine Benzyboxycarbonylgruppe oder eine Pyridylmethyboxycarbonylgruppe oder ein Salz hiervon bedeutet.

2. Verwendung eines Thiazolinderivats nach Anspruch 1 für die Herstellung von präventiven oder therapeutischen Mitteln für ischämische Erkrankungen und atherosklerotische Erkrankungen, oder Metastasen-inhibierende Mittel für bösartige Tumore.

3. Verfahren für die Herstellung des Thiazolinderivats nach Anspruch 1, umfassend die folgenden Stufen:
(i) Umsetzen einer Verbindung, dargestellt durch Formel (III) 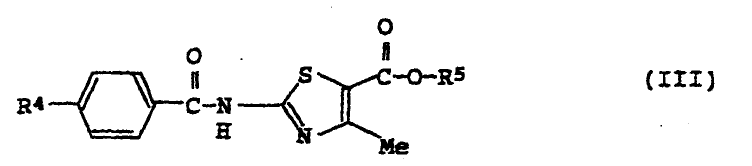 worin R⁴ eine Cyanogruppe oder eine Morpholinothiocarbonylgruppe ist, und R⁵ eine Niedrigalkylgruppe, eine Benzylgruppe oder eine 2-Cyanoethylgruppe ist, mit einem Halogenid, dargestellt durch die Formel:
R²-X
worin R² wie in Anspruch 1 definiert ist, und X für ein Halogenatom steht, in Gegenwart einer Base, wodurch die durch Formel IV dargestellte Verbindung erhalten wird 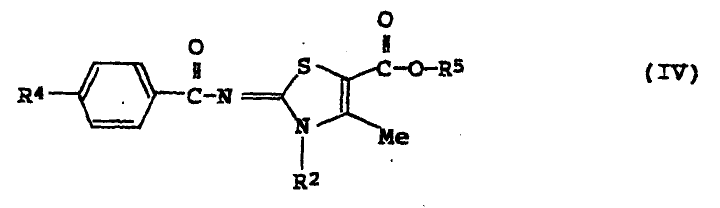 worin R², R⁴ und R⁵ wie oben definiert sind,
oder durch Umsetzen einer durch Formel (III) dargestellten Verbindung, wie oben definiert, mit einem alkylierenden Mittel, dargestellt durch die Formel:
R⁶₂-SO₄
worin R⁶ wie R², mit Ausnahme einer Aralkylgruppe, ist, oder ein Sulfonat, dargestellt durch die Formel:
R⁷SO₃R⁶
worin R⁷ eine Alkylgruppe oder eine Arylgruppe ist, und R⁶ wie oben definiert ist, um eine Verbindung der Formel IV zu erhalten 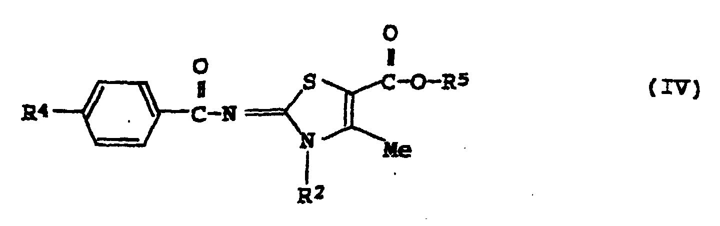 worin R² etwas anderes als eine Aralkylgruppe darstellt;
(ii) Hydrolysieren des Esterrestes der Verbindung der Formel (IV), um eine Verbindung zu erhalten, dargestellt durch die Formel: 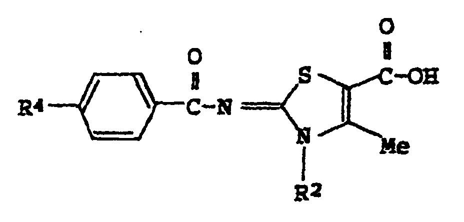 worin R² und R⁴ wie oben definiert sind oder Salze davon;
(iii) Umsetzen dieser durch Stufe (ii) erhaltenen Verbindung mit einem Amin, dargestellt durch die Formel:
H₂N-(CH₂)ₙ-R³
worin n und R³ wie in Anspruch 1 definiert sind, um ein Thiazolinderivat nach Anspruch 1 zu erhalten, worin R¹ eine Cyanogruppe oder eine Morpholinothiocarbonylgruppe ist.

4. Verfahren zur Herstellung eines Thiazolinderivats nach Anspruch 3, worin R¹ eine Thiocarbamoylgruppe ist, welches es beinhaltet, das Thiazolinderivat, welches durch die folgende Formel dargestellt wird: 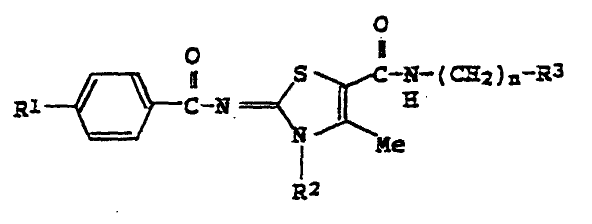 worin R¹ eine Cyanogruppe ist, und R², R³ und n wie in Anspruch 1 definiert sind, einer Reaktion mit Hydrogensulfit unter Verwendung einer Base als Katalysator oder einer Reaktion mit
NaBH₂S₃
zu unterwerfen.

5. Verfahren für die Herstellung eines Thiazolinderivats nach Anspruch 3, worin R¹ eine Carbamoylgruppe ist, welches das Umsetzen eines Thiazolinderivats, dargestellt durch die Formel: 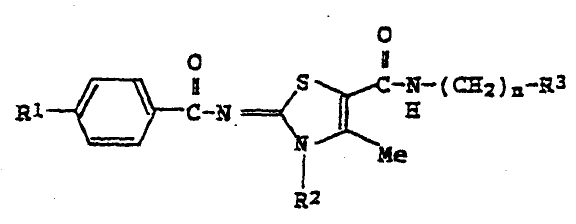 worin R¹ eine Cyanogruppe ist, und R², R³ und n wie in Anspruch 1 definiert sind, mit Wasserstoffperoxid in Gegenwart einer Base umfaßt.

6. Verfahren für die Herstellung eines Thiazolinderivats nach Anspruch 3, worin R¹ eine Alkylthioimidoylgruppe mit 2 bis 7 Kohlenstoffatomen ist, welches es umfaßt, ein Thiazolinderivat, dargestellt durch die folgende Formel: 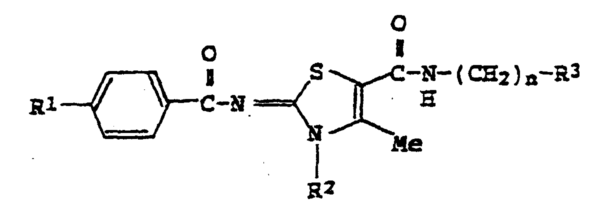 worin R¹ eine Thiacarbamoylgruppe ist, und R², R³ und n wie in Anspruch 1 definiert sind, mit einem Alkylhalogenid, dargestellt durch die Formel:
R⁸-X
worin R⁸ eine Niedrigalkylgruppe und X ein Halogenatom ist, zu behandeln.

7. Verfahren zur Herstellung eines Thiazolinderivats nach Anspruch 3, worin R¹ eine Gruppe der Formel (II) ist 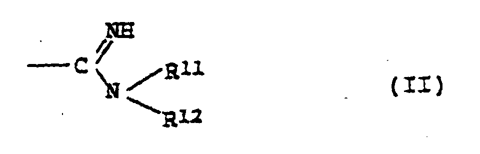 worin R¹¹ oder R¹² etwas anderes als eine Alkoxycarbonylgruppe mit 2 bis 7 Kohlenstoffatomen oder eine Imidazolin-2-yl-Gruppe darstellen, welches es umfaßt, ein Thiazolinderivat, dargestellt durch die folgende Formel: 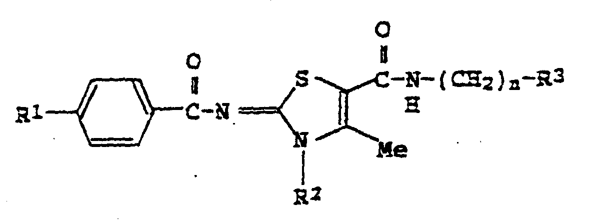 worin R¹ eine Alkylthioimidoylgruppe mit 2 bis 7 Kohlenstoffatomen ist, mit Ammoniak, einem Monoalkylamin mit 1 bis 6 Kohlenstoffatomen, einem Dialkylamin mit 1 bis 6 Kohlenstoffatomen, einem Monocycloalkylamin mit 4 bis 8 Kohlenstoffatomen, Anilin, einem substituierten Anilin, einem Aralkylamin, einem substituierten Aralkylamin, einer heterocyclischen Verbindung mit einem oder mehreren sekundären Stickstoffatomen im Ring, mit 1,2-Diaminoethan oder mit einem Salz davon umzusetzen.

8. Verfahren zur Herstellung eines Thiazolinderivats nach Anspruch 3, worin R¹ eine Gruppe der Formel (II) ist, R¹¹ und/oder R¹² eine Alkoxycarbonylgruppe mit 2 bis 7 Kohlenstoffatomen sind, welches es umfaßt, eine Verbindung, erhalten durch ein Verfahren nach Anspruch 7,
worin R¹ eine Gruppe der Formel (II) ist und R¹¹ und R¹² etwas anderes als eine Alkoxycarbonylgruppe mit 2 bis 7 Kohlenstoffatomen darstellen, mit einer Verbindung, dargestellt durch die Formeln:
R⁹-O(C=O)-X
oder
R⁹-O(C=O)-O-(C=O)O-R⁹
worin R⁹ eine Niedrigalkylgruppe und X ein Halogenatom ist, in Gegenwart einer Base umzusetzen.

9. Verfahren zur Herstellung eines Thiazolinderivats nach Anspruch 3, worin R³ eine Carboxylgruppe oder ein Salz davon ist, welches eine Esterhydrolyse durch alkalische Behandlung oder Mineralsäurebehandlung der Verbindung der Formel (I) umfaßt, worin R³ etwas anderes darstellt als eine Carboxylgruppe einer Hydroxylgruppe.

10. Verfahren zur Herstellung eines Thiazolinderivats nach Anspruch 3, worin R³ eine Alkoxycarbonylgruppe mit 2 bis 7 Kohlenstoffatomen, eine Benzyloxycarbonylgruppe oder eine Pyridylmethyloxycarbonylgruppe ist, welches das Umsetzen einer Verbindung mit der Formel: 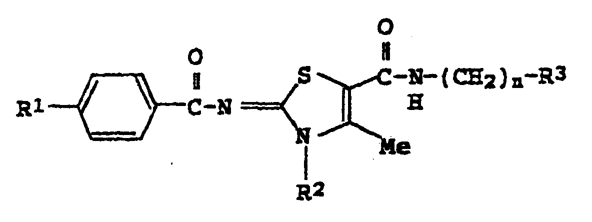 wie in Anspruch 1 beschrieben, umfaßt, worin R³ eine Carboxylgruppe oder eine Salz davon bedeutet, mit einer Verbindung, dargestellt durch die Formeln:
R¹⁰-OH
oder
R¹⁰-X
worin R¹⁰ eine Niedrigalkylgruppe, eine Benzylgruppe oder eine Pyridylmethylgruppe ist, und X eine Halogenatom ist, unter Veresterungsbedingungen.

11. Verfahren für die Herstellung eines Thiazolinderivats nach Anspruch 3, worin R³ eine Alkoxycarbonylgruppe mit 2 bis 7 Kohlenstoffatomen, eine Benzyloxycarbonylgruppe oder eine Pyridylmethyloxycarbonylgruppe ist, welches Umesterung umfaßt.

12. Verfahren für die Herstellung eines Thiazolinderivats nach Anspruch 3, worin R¹ eine Imidazolin-2-yl-Gruppe ist, durch Umsetzen einer Verbindung, dargestellt durch die Formel: 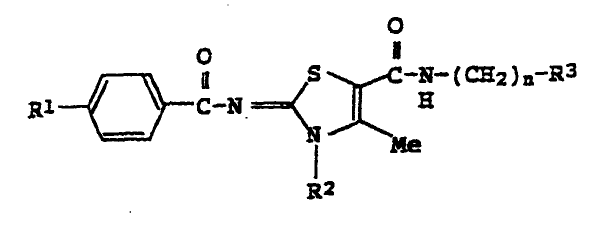 worin R¹ eine Morpholinothiocarbonylgruppe ist, und R², R³ und n so definiert sind wie in Anspruch 1, mit einem Niedrigalkylhalogenid, dargestellt durch die Formel:
R⁸-X
worin R⁸ eine Niedrigalkylgruppe, und X ein Halogenatom ist, und Umsetzen der resultierenden Verbindung mit 1,2-Diaminoethan oder einem Salz davon.

## Revendications (Revendications pour l'(les) Etat(s) contractant(s) suivant(s): AT, BE, CH, LI, DE, DK, FR, GB, IE, IT, LU, MC, NL, PT, SE)

1. Dérivé de thiazoline représenté par la formule : 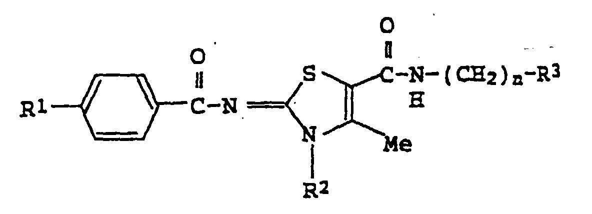 dans laquelle R¹ est un groupe cyano, un groupe carbamoyle, un groupe thiocarbamoyle, un groupe morpholinothiocarbonyle, un groupe alkylthioimidoyle ayant 2 à 7 atomes de carbone, un groupe représenté par la formule : 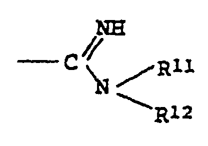 dans laquelle R¹¹ et R¹² sont identiques ou différents et sont chacun un atome d'hydrogène, un groupe alkyle ayant 1 à 6 atomes de carbone, un groupe alkcoxycarbonyle ayant 2 à 7 atomes de carbone, un groupe cycloalkyle ayant 4 à 8 atomes de carbone, un groupe phényle, un groupe phényle substitué par un groupe alkyle ayant 1 à 4 atomes de carbone, un groupe alcoxy ayant 1 à 4 atomes de carbone ou un atome d'halogène, un groupe aralkyle qui est un groupe alkyle ayant 1 à 3 atomes de carbone substitué par un groupe phényle ou un groupe naphtyle à l'extrémité de celui-ci, ou le groupe aralkyle substitué par un groupe alkyle ayant 1 à 4 atomes de carbone, un groupe alcoxy ayant 1 à 4 atomes de carbone, un groupe trifluorométhyle ou un atome d'halogène ou R¹¹ et R¹² avec l'atome d'azote adjacent forment un groupe univalent de type hétérocycle ayant 4 à 7 chaînons ayant au moins un atome d'azote sur le cycle qui peut avoir un substituant choisi parmi un alkyle, un phényle ou un benzyle en position 4 ou un groupe imidazolin-2-yle, n est un nombre entier de 1 à 3, R² est un groupe alkyle ayant 1 à 10 atomes de carbone ou un groupe aralkyle qui est un groupe alkyle ayant 1 à 3 atomes de carbone substitué par un groupe phényle ou un groupe naphtyle à l'extrémité de celui-ci, et R³ est un groupe carboxyle, un groupe hydroxyle, un groupe alcoxycarbonyle ayant 2 à 7 atomes de carbone, un groupe benzyloxycarbonyle ou un groupe pyridylméthyloxycarbonyle ou un sel de celui-ci.

2. Utilisation d'un dérivé de thiazoline tel que revendiqué dans la revendication 1, pour la préparation d'agents préventifs ou thérapeutiques pour les maladies ischémiques ou les maladies de l'athérosclérose, ou d'agents inhibiteurs des métastases pour les tumeurs malignes.

## Revendications (Revendications pour l'(les) Etat(s) contractant(s) suivant(s): GR, ES)

1. Dérivé de thiazoline représenté par la formule : 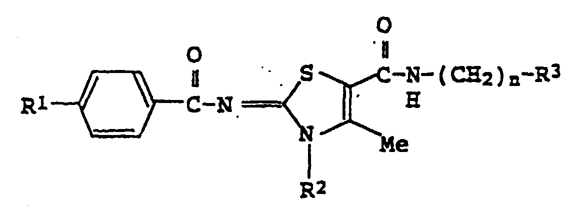 dans laquelle R¹ est un groupe cyano, un groupe carbamoyle, un groupe thiocarbamoyle, un groupe morpholinothiocarbonyle, un groupe alkylthioimidoyle ayant 2 à 7 atomes de carbone, un groupe représenté par la formule : 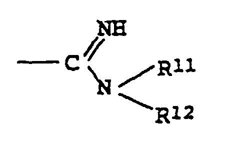 dans laquelle R¹¹ et R¹² sont identiques ou différents et sont chacun un atome d'hydrogène, un groupe alkyle ayant 1 à 6 atomes de carbone, un groupe alkcoxycarbonyle ayant 2 à 7 atomes de carbone, un groupe cycloalkyle ayant 4 à 8 atomes de carbone, un groupe phényle, un groupe phényle substitué par un groupe alkyle ayant 1 à 4 atomes de carbone, un groupe alcoxy ayant 1 à 4 atomes de carbone ou un atome d'halogène, un groupe aralkyle qui est un groupe alkyle ayant 1 à 3 atomes de carbone substitué par un groupe phényle ou un groupe naphtyle à l'extrémité de celui-ci, ou le groupe aralkyle substitué par un groupe alkyle ayant 1 à 4 atomes de carbone, un groupe alcoxy ayant 1 à 4 atomes de carbone, un groupe trifluorométhyle ou un atome d'halogène ou R¹¹ et R¹² avec l'atome d'azote adjacent forment un groupe univalent de type hétérocycle ayant 4 à 7 chaînons ayant au moins un atome d'azote sur le cycle qui peut avoir un substituant choisi parmi un alkyle, un phényle ou un benzyle en position 4 ou un groupe imidazolin-2-yle, n est un nombre entier de 1 à 3, R² est un groupe alkyle ayant 1 à 10 atomes de carbone ou un groupe aralkyle qui est un groupe alkyle ayant 1 à 3 atomes de carbone substitué par un groupe phényle ou un groupe naphtyle à l'extrémité de celui-ci, et R³ est un groupe carboxyle, un groupe hydroxyle, un groupe alcoxycarbonyle ayant 2 à 7 atomes de carbone, un groupe benzyloxycarbonyle ou un groupe pyridylméthyloxycarbonyle ou un sel de celui-ci.

2. Utilisation d'un dérivé de thiazoline tel que revendiqué dans la revendication 1, pour la préparation d'agents préventifs ou thérapeutiques pour les maladies ischémiques ou les maladies de l'athérosclérose, ou d'agents inhibiteurs des métastases pour les tumeurs malignes.

3. Procédé pour la préparation d'un dérivé de thiazoline tel que revendiqué dans la revendication 1, comprenant les étapes suivantes :
(i) faire réagir un composé représenté par la formule (III) 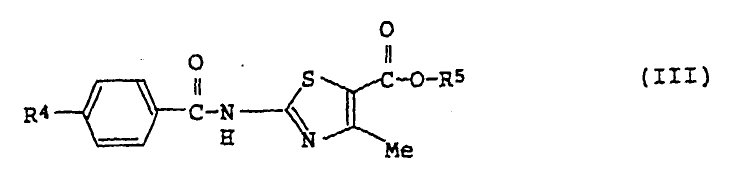 dans laquelle R⁴ est un groupe cyano ou un groupe morpholinothiocarbonyle, et R⁵ est un groupe alkyle inférieur, un groupe benzyle ou un groupe 2-cyanoéthyle avec un halogénure représenté par la formule :
R²-X
dans laquelle R² est tel que défini dans la revendication 1, et X est un atome d'halogène en présence d'une base pour donner un composé représenté par la formule IV : 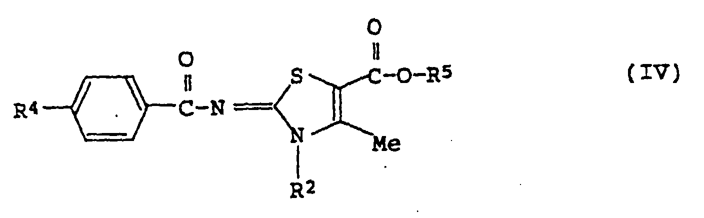 dans laquelle R², R⁴ et R⁵ sont comme définis ci-dessus,
ou faire réagir un composé représenté par la formule (III) comme défini ci-dessus avec un agent d'alkylation représenté par la formule
R⁶₂-SO₄
dans laquelle R⁶ est R² autre que un groupe aralkyle, ou un sulfonate représenté par la formule :
R⁷SO₃R⁶
dans laquelle R⁷ est un groupe alkyle ou un groupe aryle, et R⁶ est comme défini ci-dessus pour obtenir un composé de formule IV : 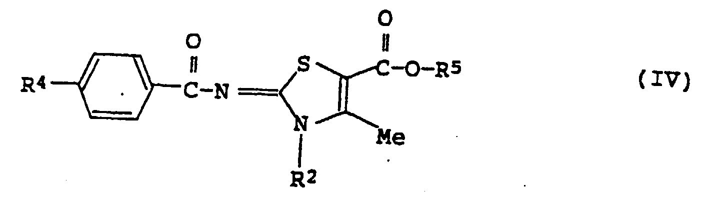 dans laquelle R² est autre qu'un groupe aralkyle;
(ii) hydrolyser le fragment ester du composé de formule (IV) pour obtenir un composé représenté par la formule : 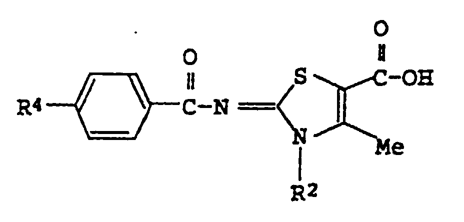 dans laquelle R² et R⁴ sont comme définis ci-dessus ou un sel de celui-ci;
(iii) faire réagir ledit composé obtenu dans l'étape (ii) avec une amine représentée par la formule :
H₂N-(CH₂)ₙ-R³
dans laquelle n et R³ sont comme définis dans la revendication 1 pour obtenir un dérivé de thiazoline tel que revendiqué dans la revendication 1, dans lequel R¹ est un groupe cyano ou un groupe morpholinothiocarbonyle.

4. Procédé pour la préparation d'un dérivé de thiazoline tel que revendiqué dans la revendication 3, dans lequel R¹ est un groupe thiocarbamoyle, qui comprend le fait de soumettre un dérivé de thiazoline représenté par la formule : 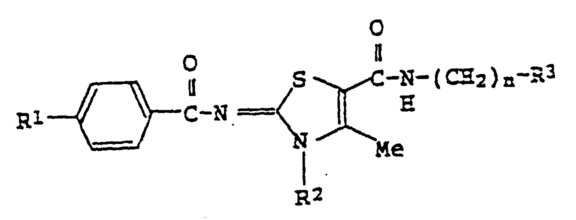 dans laquelle R¹ est un groupe cyano, et R², R³ et n sont tels que définis dans la revendication 1 à une réaction avec le sulfure d'hydrogène en utilisant une base comme catalyseur ou à une réaction avec
NaBH₂S₃.

5. Procédé pour la préparation d'un dérivé de thiazoline tel que revendiqué dans la revendication 3, dans lequel R¹ est un groupe carbamoyle, qui comprend le fait de faire réagir un dérivé de thiazoline représenté par la formule : 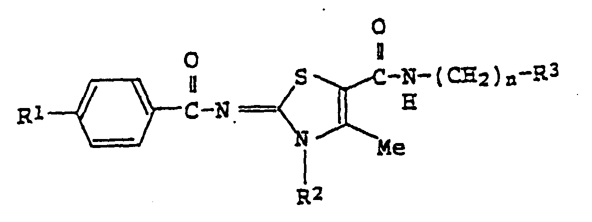 dans laquelle R¹ est un groupe cyano, et R², R³ et n sont tels que définis dans la revendication 1 avec du peroxyde d'hydrogène en présence d'une base.

6. Procédé pour la préparation d'un dérivé de thiazoline tel que revendiqué dans la revendication 3, dans lequel R¹ est un groupe alkylthioimidoyle ayant 2 à 7 atomes de carbone, qui comprend le fait de traiter un dérivé de thiazoline représenté par la formule : 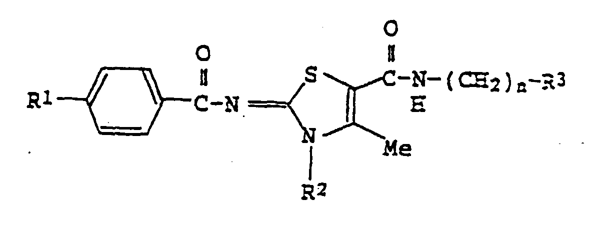 dans laquelle R¹ est un groupe thiocarbamoyle, et R², R³ et n sont comme définis dans la revendication 1 avec un halogénure d'alkyle représenté par la formule :
R⁸-X
dans laquelle R⁸ est un groupe alkyle inférieur, et X est un atome d'halogène.

7. Procédé pour la préparation d'un dérivé de thiazoline tel que revendiqué dans la revendication 3, dans lequel R¹ est un groupe de formule (II) 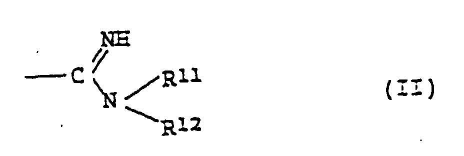 dans lequel R¹¹ ou R¹² est autre qu'un groupe alcoxycarbonyle ayant 2 à 7 atomes de carbone ou un groupe imidazolin-2-yle, qui comprend le fait de faire réagir un dérivé de thiazoline représenté par la formule : 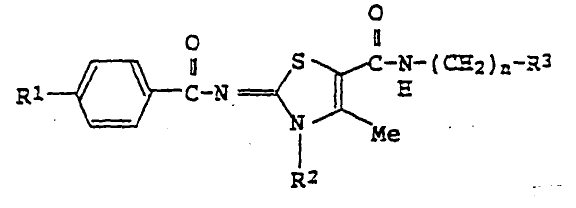 dans laquelle R² est un groupe alkylthioimidoyle ayant 2 à 7 atomes de carbone avec de l'ammoniaque, une monoalkylamine ayant 1 à 6 atomes de carbone, une dialkylamine ayant 1 à 6 atomes de carbone, une monocycloalkylamine ayant 4 à 8 atomes de carbone, une aniline, une aniline substituée, une aralkylamine, une aralkylamine substituée, un compose hétérocyclique ayant un ou plusieurs atomes d'azote secondaire sur le cycle, un 1,2-diaminoéthane ou un sel de celui-ci.

8. Procédé pour la préparation d'un dérivé de thiazoline tel que revendiqué dans la revendication 3, dans lequel R¹ est un groupe de formule (II), R¹¹ et/ou R¹² est un groupe alcoxycarbonyle ayant 2 à 7 atomes de carbone, qui comprend le fait de faire réagir un composé obtenu par le procédé selon la revendication 7, dans lequel R¹ est un groupe de formule (II), et R¹¹ et R¹² sont autres qu'un groupe alcoxycarbonyle ayant 2 à 7 atomes de carbone avec un composé représenté par la formule :
R⁹-O(C=O)-X
ou
R⁹-O(=O)-O-(C=O)O-R⁹
dans laquelle R⁹ est un groupe alkyle inférieur, et X est un atome d'halogène en présence d'une base.

9. Procédé pour la préparation d'un dérivé de thiazoline tel que revendiqué dans la revendication 3, dans lequel R³ est un groupe carboxyle ou un sel de celui-ci, qui comprend une hydrolyse de l'ester du composé de formule (I), dans lequel R³ est autre qu'un groupe carboxyle d'un groupe hydroxyle par un traitement avec un agent alcalin ou par un traitement avec un acide minéral.

10. Procédé pour la préparation d'un dérivé de thiazoline tel que revendiqué dans la revendication 3, dans lequel R³ est un groupe alcoxycarbonyle ayant 2 à 7 atomes de carbone, un groupe benzyloxycarbonyle ou un groupe pyridylméthyloxycarbonyle, qui comprend le fait de faire réagir un composé de formule : 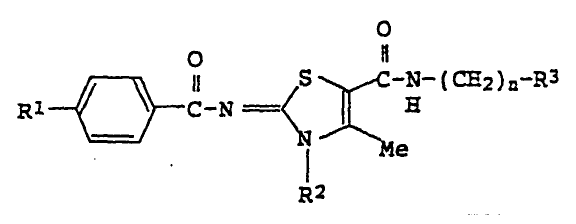 tel que défini dans la revendication 1, dans lequel R³ est un groupe carboxyle ou un sel de celui-ci avec un composé représenté par la formule :
R¹⁰-OH
ou
R¹⁰-X
dans laquelle R¹⁰ est un groupe alkyle inférieur, un groupe benzyle ou un groupe pyridylméthyle, et X est un atome d'halogène dans des conditions d'estérification.

11. Procédé pour la préparation d'un dérivé de thiazoline tel que revendiqué dans la revendication 3, dans lequel R³ est un groupe alcoxycarbonyle ayant 2 à 7 atomes de carbone, un groupe benzyloxycarbonyle ou un groupe pyridylméthyloxycarbonyle, qui comprend un échange d'ester.

12. Procédé pour la préparation d'un dérivé de thiazoline tel que revendiqué dans la revendication 3, dans lequel R¹ est un groupe imidazolin-2-yle par réaction d'un composé représenté par la formule : 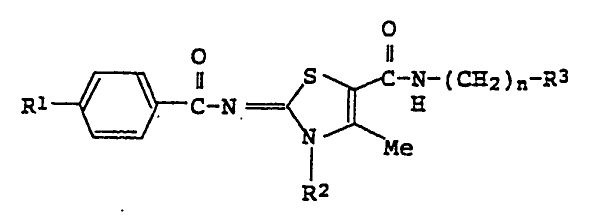 dans laquelle R¹ est un groupe morpholinothiocarbonyle, et R², R³ et n sont comme définis dans la revendication 1 avec un halogénure d'alkyle inférieur représenté par la formule:
R⁸-X
dans laquelle R⁸ est un groupe alkyle inférieur, et X est un atome d'halogène et en faisant réagir le composé résultant avec du 1,2-diaminoéthane ou un sel de celui-ci.
